# EUROPEAN PATENT APPLICATION

(11) **EP 4 180 515 A1**
(43) Date of publication of application: **17.05.2023**
(21) Application number: 21858309.4
(22) Date of filing: 17.08.2021
(51) Int. Cl.: C12N 5/071, C12N 5/073, C12N 5/074, C12N 5/0775, C12N 5/078, C12N 5/10, C12N 15/12

(54) **METHOD FOR INDUCING DIFFERENTIATION OF PLURIPOTENT STEM CELLS INTO ECTODERMAL, MESODERMAL, AND ENDODERMAL CELLS**

(30) Priority: 18.08.2020 JP 2020138106; 27.04.2021 JP 2021075272
(71) Applicant: Tosoh Corporation, Yamaguchi 746-8501 (JP)
(72) Inventor: KADOTA Junpei, Yokkaichi-shi, Mie 510-8540 (JP); IMAIZUMI Yu, Yokkaichi-shi, Mie 510-8540 (JP)
(74) Representative: Novagraaf International SA
(86) International application number: PCT/JP2021/030058
(87) International publication number: WO 2022/039165

(57) **Abstract**

The present invention relates to a method for inducing differentiation of a pluripotent stem cell into an ectodermal cell, the method comprising steps (1-1) to (1-4) below: (1-1) a step of coating a cell culture substrate comprising two regions (A) and (B) below with a single or a plurality of substances selected from a group consisting of Matrigel or the like, and seeding the pluripotent stem cell, (A) an island-shaped region with an area of 0.001 to 5 mm² having cell adhesiveness or the like, and (B) a region adjacent to the region (A) and having no cell adhesiveness or the like. (1-2) a step of forming a pluripotent stem cell aggregate adhering onto the cell culture substrate by adherently culturing the pluripotent stem cell seeded in the step (1-1) in a feeder-free manner in the presence of an undifferentiated state maintenance medium; (1-3) a step of forming an embryoid body by culturing the pluripotent stem cell aggregate formed in the step (1-2) in the presence of a medium comprising a differentiation-inducing factor in a state of adhering onto the cell culture substrate; and (1-4) a step of forming an ectodermal cell aggregate by culturing the embryoid body formed in the step (1-3) in the presence of a medium comprising a differentiation-inducing factor in a state of adhering onto the cell culture substrate.

## Description

### Technical Field

The present invention provides a method for inducing differentiation of pluripotent stem cells into ectodermal cells and a method for producing ectodermal cells, a method for inducing differentiation of pluripotent stem cells into mesodermal cells, and a method for inducing differentiation of pluripotent stem cells into endodermal cells, which are excellent in differentiation induction efficiency and culture operability.

### Background Art

There is growing interest in drug discovery using nerve cells induced to differentiate from induced pluripotent stem cells (iPS cells) for neurodegenerative diseases such as amyotrophic lateral sclerosis (ALS). There is also a growing interest in drug discovery using endodermal cells such as liver cells, small intestinal cells, and pancreatic cells, cardiomyocytes, skeletal muscle cells, or vascular endothelial cells, which are induced to differentiate from induced pluripotent stem cells (iPS cells). The use of iPS cells derived from diseased patients is expected to contribute to the elucidation of disease mechanisms, drug discovery research, and development of regenerative medicine, which have been difficult in the past. In general, in the differentiation induction into nerve cells, differentiation induction into each peripheral nerve cell is performed according to the step of forming pluripotent stem cell aggregates called embryoid bodies (embryoids), the step of forming neural stem cell aggregates called neurospheres, and the like. In general, in the differentiation induction into the mesodermal cells, the differentiation induction into tissues derived from each mesodermal cell is performed according to the step of forming pluripotent stem cell aggregates called embryoid bodies (embryoids). In general, in the differentiation induction into the endodermal cells, the differentiation induction into tissues derived from each endodermal cell is performed through the formation of pluripotent stem cell aggregates called embryoids. In addition, it is known that the state of embryoid bodies (embryoids) in the process of differentiation induction into target cells greatly contributes to the fate of cells such as differentiation pathways, and the problem is how to efficiently prepare uniform embryoid bodies (embryoids).

In conventional methods, embryoid bodies (embryoids) and neurospheres are prepared by floating culture methods such as hanging drop culture (for example, Patent Literature 1) and non-adhesive surface culture (for example, Patent Literature 2) (for example, Patent Literature 3).

As another method using the floating culture method, there is a method that utilizes the formation of cell aggregates according to the size of unevenness by seeding cells on a non-adhesive cell substrate with a fine uneven structure on the surface thereof and allowing the cells to settle (for example, Patent Literature 4).

Patent Literature 5 discloses a cell culture substrate that enables efficient spheroid formation of cells, has a high survival rate of cells, and is capable of forming spheroids of uniform size and any shape, a method for producing the cell culture substrate, and a method for producing spheroids with excellent cell viability inside the spheroids using the cell culture substrate.

Patent Literature 6 describes that ES cells were seeded on a substrate coated with 0.1% human recombinant type I collagen peptide or vitronectin to perform differentiation induction and to prepare organoids that perform peristalsis similar to the intestine.

### Citation List

### Patent Literature

[Patent Literature 1] WO 2008/001938
[Patent Literature 2] WO 2017/123791
[Patent Literature 3] JP 2002-291469
[Patent Literature 4] WO 2018/123663
[Patent Literature 5] JP 2020-62009
[Patent Literature 6] JP 2019-14

### Summary of Invention

### Technical Problem

In the culture methods described in Patent Literature 1 to 3, aggregates to be formed are non-uniform in size, and thus there is a problem that the differentiation induction efficiency tends to vary between individuals. In addition, since aggregates of different sizes are arranged three-dimensionally, there is a risk of local nutritional deficiencies. In the method described in Patent Literature 4, since the aggregates are not immobilized on the substrate surface, some of the aggregates are removed together with the medium when the medium is exchanged, and there is a problem that the culture operation requires proficiency. In addition, due to the nature of the substrate, it is difficult to exchange the entire amount of the medium, and thus nutrient deficiencies such as differentiation-inducing factors are likely to occur, and there is a concern that the differentiation induction efficiency will be inferior. In the culture method described in Patent Literature 6, the organoids to be formed are spontaneously dissociated from the substrate approximately 30 days after initiation of differentiation induction. When the organoids are dissociated, there is a problem that handling is difficult during culture operations or immunostaining for cell function evaluation.

An object of the present invention is to provide a method for inducing differentiation of pluripotent stem cells into ectodermal cells and a method for producing the ectodermal cells, which are excellent in differentiation induction efficiency and culture operability.

Another object of the present invention is to provide a method for inducing differentiation of pluripotent stem cells into mesodermal cells, which is excellent in differentiation induction efficiency and culture operability.

Still another object of the present invention is to provide a method for inducing differentiation of pluripotent stem cells into endodermal cells, which is excellent in simplicity of culture operations such as medium exchange and cell observation, and is also excellent in operability during cell function evaluation such as immunostaining and secretion measurement tests.

### Solution to Problem

The present inventors have found that the above problems can be solved by forming embryoid bodies (embryoids) adhering onto the cell culture substrate by seeding pluripotent stem cells on the cell culture substrate having cell adhesion regions formed in the shape of islands, and by inducing differentiation of adherent embryoid bodies (embryoids), and have completed the present invention.

That is, according to one aspect of the present invention, there is provided a method for inducing differentiation of pluripotent stem cells into ectodermal cells and a method for producing ectodermal cells, the method including steps (1-1) to (1-4) below.
<1> A method for inducing differentiation of a pluripotent stem cell into an ectodermal cell, the method including steps (1-1) to (1-4) below. (1-1) A step of coating a cell culture substrate comprising two regions (A) and (B) below with a single or a plurality of substances selected from a group consisting of Matrigel, laminin, fibronectin, vitronectin, and collagen, and seeding the pluripotent stem cell. (A) An island-shaped region with an area of 0.001 to 5 mm² having cell adhesiveness and cell proliferation. (B) A region adjacent to the region (A) and having no cell adhesiveness or having no cell proliferation. (1-2) A step of forming a pluripotent stem cell aggregate adhering onto the cell culture substrate by adherently culturing the pluripotent stem cell seeded in the step (1-1) in a feeder-free manner in the presence of an undifferentiated state maintenance medium. (1-3) A step of forming an embryoid body (embryoid) by culturing the pluripotent stem cell aggregate formed in the step (1-2) in the presence of a medium comprising a differentiation-inducing factor in a state of adhering onto the cell culture substrate. (1-4) A step of forming an ectodermal cell aggregate by culturing the embryoid body (embryoid) formed in the step (1-3) in the presence of a medium comprising a differentiation-inducing factor in a state of adhering onto the cell culture substrate.
<2> The method for inducing differentiation according to <1>, wherein the cell culture substrate has a layer comprising a hydrophilic polymer on a surface thereof, and the region (A) is a region obtained by decomposing or modifying a part of the layer comprising the hydrophilic polymer by plasma treatment, ultraviolet treatment, corona discharge treatment, or a combination thereof.
<3> The method for inducing differentiation according to <1> or <2>, wherein the differentiation-inducing factor in the step (1-3) comprises an ectoderm-inducing factor, a mesoderm-inducing factor, and an endoderm-inducing factor.
<4> The method for inducing differentiation according to any one of <1> to <3>, wherein the ectodermal cell aggregate formed in the step (1-4) comprise PAX6 and SOX1.
<5> The method for inducing differentiation according to any one of <1> to <4>, wherein the ectodermal cell is a neural stem cell or a nerve cell.
<6> The method for inducing differentiation according to any one of <1> to <5>, wherein the ectodermal cell is a motor nerve cell.
<7> A method for producing a cell obtained by differentiation induction of a pluripotent stem cell into an ectodermal cell, the method including steps (1-1) to (1-4) below. (1-1) A step of coating a cell culture substrate comprising two regions (A) and (B) below with a single or a plurality of substances selected from a group consisting of Matrigel, laminin, fibronectin, vitronectin, and collagen, and seeding the pluripotent stem cell. (A) An island-shaped region with an area of 0.001 to 5 mm² having cell adhesiveness and cell proliferation. (B) A region adjacent to the region (A) and having no cell adhesiveness or having no cell proliferation. (1-2) A step of forming a pluripotent stem cell aggregate adhering onto the cell culture substrate by adherently culturing the pluripotent stem cell seeded in the step (1-1) in a feeder-free manner in the presence of an undifferentiated state maintenance medium. (1-3) A step of forming embryoid bodies (embryoids) by culturing the pluripotent stem cell aggregate formed in the step (1-2) in the presence of a medium comprising a differentiation-inducing factor in a state of adhering onto a cell culture substrate. (1-4) A step of forming an ectodermal cell aggregate by culturing the embryoid body (embryoid) formed in the step (1-3) in the presence of a medium comprising a differentiation-inducing factor in a state of adhering onto the cell culture substrate.

The present invention includes each aspect of [1] to [8] shown below.
[1] A method for inducing differentiation of a pluripotent stem cell into a mesodermal cell, the method including steps (2-1) to (2-3) below. (2-1) A step of coating a cell culture substrate comprising two regions (A) and (B) below with a single or a plurality of substances selected from a group consisting of Matrigel, laminin, fibronectin, vitronectin, and collagen, and seeding the pluripotent stem cell.
   (A) An island-shaped region with an area of 0.001 to 5 mm² having cell adhesiveness and cell proliferation.
   (B) A region adjacent to the region (A) and having no cell adhesiveness or having no cell proliferation. (2-2) A step of forming an embryoid body (embryoid) adhering onto the cell culture substrate by adherently culturing the pluripotent stem cell seeded in the step (2-1) in a feeder-free manner in the presence of an undifferentiated state maintenance medium. (2-3) A step of forming a mesodermal cell aggregate by culturing the embryoid body (embryoid) formed in the step (2-2) in the presence of a medium comprising a differentiation-inducing factor in a state of adhering onto the cell culture substrate.
[2] The method for inducing differentiation according to [1], wherein the cell culture substrate has a layer comprising a hydrophilic polymer on a surface thereof, and the region (A) is a region obtained by decomposing or modifying the layer comprising the hydrophilic polymer by plasma treatment, ultraviolet treatment, and/or corona discharge treatment.
[3] The method for inducing differentiation according to [1] or [2], wherein a cell seeding density in the step (2-1) is 1.0 × 10² to 1.0 × 10⁶ cells/cm².
[4] The method for inducing differentiation according to any one of [1] to [3], wherein, in the step (2-2), the embryoid body (embryoid) is cultured until the number of cells per unit area of the region (A) reaches 1.0 × 10⁴ cells/cm² or more.
[5] The method for inducing differentiation according to any one of [1] to [4], wherein a shape of the embryoid body (embryoid) formed in the step (2-2) is an island shape.
[6] The method for inducing differentiation according to any one of [1] to [5], wherein the differentiation-inducing factor in the step (2-3) comprises a mesoderm-inducing factor.
[7] The method for inducing differentiation according to [6], wherein the mesoderm-inducing factor is single or a plurality of differentiation-inducing factors selected from a group consisting of a GSK3β inhibitor, bone morphogenetic protein (BMP), and activin.
[8] The method for inducing differentiation according to any one of [1] to [7], wherein the embryoid body (embryoid) formed in the step (2-2) comprises a mesoderm marker.

According to another aspect of the present invention, there is provided a method for inducing differentiation of a pluripotent stem cell into an endodermal cell, the method including:
(3-1) a step of adding a composition comprising at least one selected from laminin and a fragment thereof to a cell culture substrate comprising a region (A) below and a region (B) below based on an area of a culture surface of the cell culture substrate such that a total amount of laminin and the fragment thereof is 1 to 100 µg/cm², and seeding the pluripotent stem cell,
   (A) an island-shaped region with an area of 0.001 to 5 mm² having cell adhesiveness and cell proliferation, and
   (B) a region adjacent to the region (A) and having no cell adhesiveness or having no cell proliferation;
(3-2) a step of forming an embryoid adhering onto the culture surface of the cell culture substrate by adherently culturing the pluripotent stem cell seeded in the step (3-1) in a feeder-free manner in the presence of an undifferentiated state maintenance medium; and
(3-3) a step of forming an endodermal cell aggregate by culturing the embryoid formed in the step (3-2) in the presence of a medium comprising a differentiation-inducing factor in a state of adhering onto the culture surface of the cell culture substrate, and by performing differentiation induction.

According to still another aspect of the present invention, there is provided a kit for inducing differentiation into a tridermic cell, including a cell culture substrate comprising a region (A) below and a region (B) below.
(A) an island-shaped region with an area of 0.001 to 5 mm² having cell adhesiveness and cell proliferation.
(B) a region adjacent to the region (A) and having no cell adhesiveness or having no cell proliferation.

### Advantageous Effects of Invention

According to the present invention, it is possible to provide a method for inducing differentiation and a kit for inducing differentiation of pluripotent stem cells into ectodermal cells, which are excellent in differentiation induction efficiency and culture operability. In the embryoid bodies (embryoids) induced to differentiate in a culture system immobilized on a cell culture substrate having the above-described regions (A) and (B), any shape (diameter of embryoid bodies (embryoids), aspect ratio of embryoid bodies (embryoids)) can be maintained, the concentration gradient of the differentiation-inducing factor has little effect, and thus the differentiation progress of each cell can be synchronized. In addition, by inducing differentiation in a culture system in which cell aggregates are feeder-free and immobilized on a cell culture substrate, contamination with contaminants can be reduced.

According to the present invention, it is possible to provide a method for inducing differentiation and a kit for inducing differentiation of pluripotent stem cells into mesodermal cells, which are excellent in differentiation induction efficiency and culture operability.

According to the present invention, it is possible to provide a method for inducing differentiation and a kit for inducing differentiation of pluripotent stem cells into endodermal cells, which are excellent in simplicity of culture operations such as medium exchange and cell observation, and is also excellent in operability during cell function evaluation such as immunostaining and secretion measurement tests. According to the method for inducing differentiation and the kit for inducing differentiation according to the present invention, it is possible to efficiently form pluripotent stem cell aggregates, and to perform culturing in a consistently adherent state from differentiation induction into endodermal cells to cell function evaluation, and thus simplicity of culture operation and the operability during cell function evaluation are excellent.

### Brief Description of Drawings

FIG. 1 is a schematic diagram (sectional view) of a cell culture substrate.

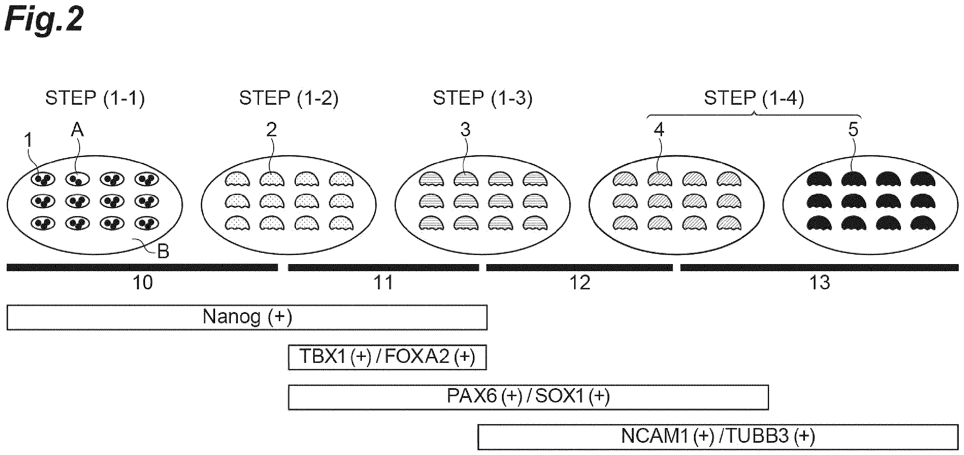

FIG. 2 is a schematic diagram of a method for inducing differentiation into ectodermal cells according to the present invention.
FIG. 3 is phase-contrast micrographs of embryoid bodies (embryoids) on a cell culture substrate in Example 1.
FIG. 4 is a graph showing temporal changes in diameter and aspect ratio of embryoid bodies (embryoids) in Example 1.

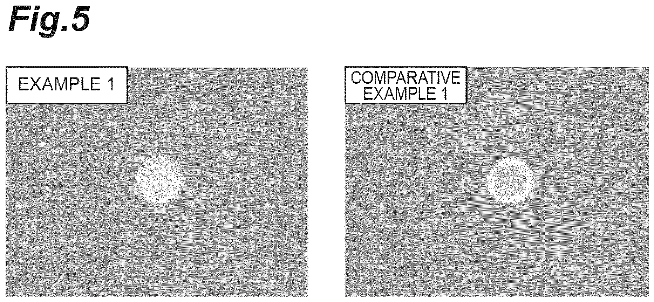

FIG. 5 is phase-contrast micrographs showing pseudopodia extension at an outer edge of an embryoid body (embryoid) in Example 1 and Comparative Example 1.
FIG. 6 is phase-contrast micrographs of embryoid bodies (embryoids) in Comparative Examples 2 and 3.

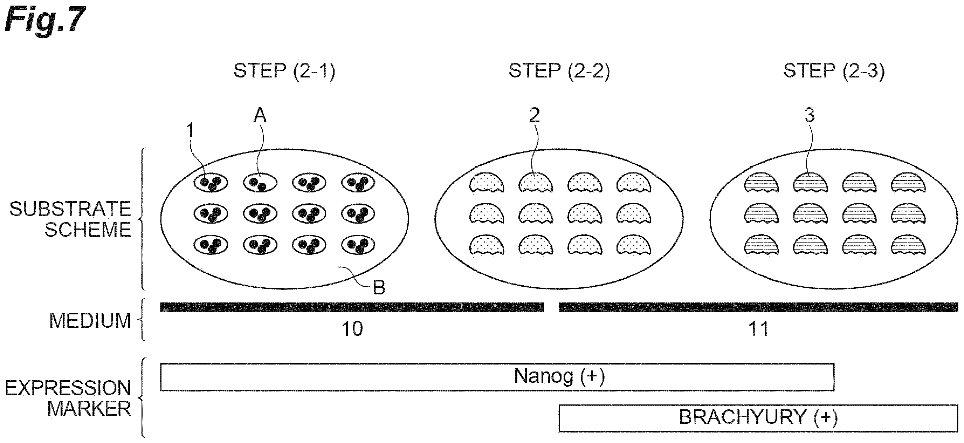

FIG. 7 is a schematic diagram showing a method for inducing differentiation into mesodermal cells according to the present invention.
FIG. 8 shows a phase-contrast micrograph of embryoid bodies (embryoids) in Example 2.
FIG. 9 shows phase-contrast micrographs in Comparative Examples 4 and 5.
FIG. 10 shows gene expression level evaluation results for a mesoderm marker (BRACHYURY) in Example 2 and Comparative Examples 4 and 5.
FIG. 11 shows gene expression level evaluation results for an undifferentiated marker (NANOG) in Example 2 and Comparative Examples 4 and 5.
FIG. 12 is a diagram showing an outline of a culture method in Example 3.
FIG. 13 is a phase-contrast micrograph of embryoids in Example 3. Scale bar indicates 100 µm.
FIG. 14 is phase-contrast micrographs of embryoids in Comparative Examples 6 and 7. Scale bar indicates 100 µm.
FIG. 15 is a graph showing evaluation results of gene expression levels of an undifferentiated marker (NANOG) and an endoderm marker (SOX17) in Example 3 and Comparative Examples 6 and 7.
FIG. 16 is phase-contrast micrographs of cells induced to differentiate taken over time in Example 4. Scale bar indicates 200 µm.
FIG. 17 is phase-contrast micrographs of cells induced to differentiate in Examples 4 and 5, and fluorescence micrographs after immunostaining. Scale bar indicates 200 µm.
FIG. 18 is phase-contrast micrographs of cells induced to differentiate in Comparative Example 8 and Example 4. Scale bar indicates 200 µm.
FIG. 19 is phase-contrast micrographs of cells induced to differentiate in Comparative Example 9. Scale bar indicates 200 µm. Arrows indicate a cell mass dissociated from the substrate.

### Description of Embodiments

Hereinafter, the mode for carrying out the present invention (hereinafter, simply referred to as "the present embodiment") will be described in detail. The following embodiments are examples for describing the present invention, and are not intended to limit the present invention to the following contents. The present invention can be appropriately modified and implemented within the scope of its gist.

In the present specification, the term "pluripotent stem cell" refers to a cell that has the property (undifferentiated state or pluripotency) of being able to differentiate into various cells. In addition, in the present specification, the term "ectodermal cells" refers to a general term for cells contained in the ectoderm formed by early embryos (ectodermal cells) or cells contained in tissues derived from the ectoderm (including neural stem cells and nerve cells).

Furthermore, the term "neural stem cells" refers to cells in which differentiation induction into neuroectoderm progresses, and differentiation induction into glial cells (astrocytes, oligodendrocytes), central nerves (dopamine nerves, GABA nerves), and peripheral nerves (motor nerves, sensory nerves) is possible. In addition, in the present specification, the term "mesodermal cell" refers to a general term for cells contained in mesoderm formed by early embryos (mesodermal cells) or cells contained in tissues derived from mesoderm. In addition, in the present specification, the term "endodermal cell" refers to a general term for cells contained in endoderm formed by early embryos (endodermal cells) or cells contained in tissues derived from endoderm. Further, in the present specification, the term "undifferentiated state maintenance" refers to a state where cultured cells have pluripotency. The method for evaluating the undifferentiated state maintenance is not particularly limited, but examples thereof include analysis of cell surface markers by alkaline phosphatase staining, analysis of cell surface/cell nuclear markers by immunostaining and flow cytometry, analysis of gene expression level by real-time RT-PCR, confirmation of embryoid body (embryoid) formation, which is a unique structure formed by pluripotent stem cells, and teratoma assay for determining in vivo trigeminal differentiation from pathological sections.

In addition, in the present specification, the term "differentiation" refers to a state where the expression of cell type-specific membrane proteins, transcription factors, and the like positioned downstream of pluripotent stem cells in the differentiation-inducing pathway of pluripotent stem cells is confirmed. Furthermore, the term "differentiation induction" refers to the progress of cell differentiation by culturing cells in the presence of a specific protein, gene, natural product, synthetic chemical substance, or the like. In addition, in the present specification, the term "cell aggregate (pluripotent stem cell aggregate)" refers to a three-dimensional structure formed by aggregation of a plurality of cells (pluripotent stem cells). Furthermore, "embryoid body (embryoid)" is a spherical cell aggregate (pluripotent stem cell aggregate) seen in the early stage of embryogenesis, and indicates characteristics of ectoderm, mesoderm, and endoderm similar to early embryos.

In addition, in the present specification, the term "marker" refers to a protein or gene specific to a particular cell. A marker possessed by pluripotent stem cells is referred to as "undifferentiated marker," a marker possessed by ectodermal cells is referred to as "ectoderm marker," a marker possessed by mesodermal cells is referred to as "mesoderm marker," a marker possessed by endodermal cells is referred to as "endoderm marker," a marker possessed by neural stem cells is referred to as "neural stem cell marker," a marker possessed by nerve cells is referred to as "nerve cell marker," and a marker possessed by small intestinal tissue cells is referred to as "intestinal epithelial cell marker," respectively. Pluripotent stem cells do not possess ectoderm, mesoderm, and endoderm markers. In addition, embryoid bodies (embryoids) also have ectoderm markers, mesoderm markers, and endoderm markers.

In addition, in the present specification, the term "cell adhesiveness" indicates easiness of adhesion to the cell culture substrate at the culture temperature, and "having cell adhesiveness" indicates that the cells can adhere to the cell culture substrate at the culture temperature. Moreover, "having no cell adhesiveness" indicates that cells cannot adhere to the cell culture substrate at the culture temperature.

In addition, in the present specification, the term "cell proliferation" indicates the easiness of cell growth at the culture temperature, and the term "having cell proliferation" indicates that the cells can proliferate at the culture temperature. Moreover, "having no cell proliferation" indicates that cells cannot proliferate at the culture temperature.

### <Method for inducing differentiation into ectodermal cells>

The method for inducing differentiation into ectodermal cells according to the present invention uses a cell culture substrate having the following two regions. (A) An island-shaped region with an area of 0.001 to 5 mm² having cell adhesiveness and cell proliferation. (B) A region adjacent to the region (A) and having no cell adhesiveness. By using the cell culture substrate having the region (A) as the cell culture substrate, pluripotent stem cells can be adherently cultured on the cell culture substrate in the step (1-1) described later, and the embryoid bodies (embryoids) adhering to the cell culture substrate can be formed in the step (1-2) described later. When there is no region (A), pluripotent stem cells cannot be adherently cultured on the cell culture substrate and embryoid bodies (embryoids) adhering to the cell culture substrate cannot be formed. In addition, by using a cell culture substrate having the region (B) as the cell culture substrate, pluripotent stem cells can proliferate only in the region (A), and uniform-sized embryoid bodies (embryoids) can be formed. Differentiation induction efficiency can be enhanced by forming uniform-sized embryoid bodies (embryoids). When there is no region (B), uniform-sized embryoid bodies (embryoids) cannot be formed.

As the area of the region (A), from the viewpoint of suitability of forming embryoid bodies (embryoids) having a size suitable for inducing differentiation into ectodermal cells, the area of the region (A) is more preferably 0.005 to 3 mm², particularly preferably 0.01 to 1.0 mm², and most preferably 0.03 to 0.8 mm².

The shape of the region (A) is not particularly limited, but is preferably a circle, an ellipse, or a regular polygon, more preferably a circle, since it is suitable for enhancing the differentiation induction efficiency.

The shape of the region (B) is not particularly limited except that the region (B) is adjacent to the region (A), but from the viewpoint of suitability of producing aggregates having a uniform size and shape, the shape of the region (B) is preferably adjacent to the length of 20% or more of the boundary line with the (A) region, more preferably 50% or more, still more preferably 80% or more, and most preferably, the entire circumference of the region (A) is the region (B). Moreover, from the viewpoint of suitability of enhancing the mass productivity of the cell culture substrate, it is preferable that the region (A) have an island shape and the region (B) have a sea-island structure.

The area ratio of the region (A) and the region (B) is not particularly limited, but from the viewpoint of increasing the number of cell aggregates that can be produced per unit area of the cell culture substrate, the area of the region (A) is preferably 10% or more, more preferably 30% or more, still more preferably 50% or more, and most preferably 70% or more of the total area of the region (A) and the region (B). In addition, from the viewpoint of suitability of providing a sufficient distance between the plurality of regions (A) and suppressing fusing of the aggregates of the plurality of regions (A) to form an uneven shape, the area of the region (B) is preferably 20% or more, more preferably 40% or more, still more preferably 60% or more, and most preferably 80% or more of the total area of the region (A) and the region (B).

The cell culture substrate used in the method for inducing differentiation of the present invention forms uniform-sized embryoid bodies (embryoids), and from the viewpoint of suitability of enhancing differentiation induction efficiency, it is preferable that the layer comprising hydrophilic polymer be contained on the surface, and the region (A) be a region in which a part of the layer comprising the hydrophilic polymer is decomposed or modified by plasma treatment, ultraviolet treatment, corona discharge treatment, or a combination thereof. By comprising a layer comprising hydrophilic polymer on the surface of the cell culture substrate, adsorption of proteins that contribute to substrate-cell adhesiveness in the region (B) is suppressed, and the region (B) can be set to a region having no cell adhesiveness, having no cell proliferation, or having no cell adhesiveness or cell proliferation.

In addition, by partially decomposing or modifying the layer comprising the hydrophilic polymer, the region (A) can be imparted with cell adhesiveness or cell proliferation. In addition, from the viewpoint of the suitability of enhancing the cell adhesiveness and cell proliferation of the region (A) and forming embryoid bodies (embryoids) in a short period of time, it is more preferable that the region (A) be a plasma-treated region.

From the viewpoint of suitability of setting the region (B) to a region having no cell adhesiveness or having no cell proliferation, the layer thickness of the layer comprising the hydrophilic polymer is preferably 10 nm or more, more preferably 50 nm or more, particularly preferably 100 nm or more, and most preferably 500 nm or more. In addition, from the viewpoint of suitability of setting the region (A) to a region having cell adhesiveness and cell proliferation, the layer thickness is preferably 1000 nm or less, more preferably 500 nm or less, particularly preferably 100 nm or less, and most preferably 50 nm or less.

At least one of a method of forming a chemical bond and a method of physical interaction can be used as a method for forming the layer comprising the hydrophilic polymer. Examples of methods for forming chemical bonds include methods for forming reactive functional groups, such as ultraviolet irradiation, electron beam irradiation, gamma ray irradiation, plasma treatment, and corona treatment. In addition, a cross-linking reaction to the substrate surface by an organic reaction using ions or radicals as a reaction source is also possible. As a method of physical interaction, methods of coating, brush coating, dip coating, spin coating, bar coating, flow coating, spray coating, roll coating, air knife coating, blade coating, gravure coating, micro gravure coating, slot die coating, and the like can be used using a matrix that is highly compatible with the target hydrophilic polymer as the coating material.

Although the type of the hydrophilic polymer is not particularly limited, examples thereof include those having a polar group such as a hydroxy group, an amino group, and a polyethylene glycol group, and those having a zwitterionic structure such as a betaine structure and a phosphorylcholine group. From the viewpoint of setting the region (B) to a region having no cell adhesiveness and cell proliferation, a hydroxy group, a phosphorylcholine group, or a polyethylene glycol group is preferable, a hydroxy group or a phosphorylcholine group is more preferable, and a phosphorylcholine group is particularly preferable.

Regarding the hydrophilic polymer, from the viewpoint of suitability of suppressing the elution of the hydrophilic polymer from the cell culture substrate, and suppressing the influence on the quality due to the contamination of the cell aggregates and the embryoid bodies (embryoids) by the polymer, a random copolymer or a block copolymer having both hydrophilic monomeric units and hydrophobic monomeric units is preferable, and a random copolymer having both hydrophilic monomeric units and hydrophobic monomeric units is more preferable. In addition, as the composition ratio of the copolymer, from the viewpoint of suitability of setting the region (B) to a region having no cell adhesiveness and cell proliferation, the hydrophilic monomeric unit is preferably 30 wt% or more, more preferably 40 wt% or more, particularly preferably 50 wt% or more, and most preferably 60 wt% or more. Furthermore, from the viewpoint of suitability of suppressing the elution of hydrophilic polymers, the hydrophobic monomeric unit is preferably 20 wt% or more, more preferably 30 wt% or more, particularly preferably 40 wt% or more, and most preferably 50 wt% or more.

The hydrophilic monomeric unit is not particularly limited except that the monomeric unit is hydrophilic, and examples thereof include: those comprising an amino group such as 2-dimethylaminoethyl acrylate, 2-dimethylaminoethyl methacrylate, 2-diethylaminoethyl acrylate, 2-diethylaminoethyl methacrylate, N-[3-(dimethylamino)propyl]acrylamide; those comprising betaine such as N-(3-sulfopropyl)-N-methacryloyloxyethyl-N,N-dimethylammonium betaine, and N-methacryloyloxyethyl-N,N-dimethylammonium-α-N-methylcarboxybetaine; those comprising a polyethylene glycol group or a methoxyethyl group such as hydroxyethyl acrylate, hydroxyethyl methacrylate, N-(2-hydroxyethyl)acrylamide, polyethylene glycol monoacrylate, polyethylene glycol monomethacrylate, polypropylene glycol monoacrylate, polypropylene glycol monomethacrylate, methoxypolyethylene glycol monoacrylate, methoxypolyethylene glycol monomethacrylate, diethylene glycol monomethyl ether acrylate, diethylene glycol monomethyl ether methacrylate, diethylene glycol monoethyl ether acrylate, diethylene glycol monoethyl ether methacrylate, 2-methoxyethyl acrylate, 2-methoxyethyl methacrylate, 2-ethoxyethyl acrylate, 2-ethoxyethyl methacrylate, 3-butoxyethyl acrylate, 3-butoxyethyl methacrylate, 3-butoxyethyl acrylamide, furfuryl acrylate, furfuryl methacrylate, tetrahydrofurfuryl acrylate, and tetrahydrofurfuryl methacrylate; those comprising an acrylate group such as methoxymethyl acrylate, methoxymethyl methacrylate, 2-ethoxymethyl acrylate, 2-ethoxymethyl methacrylate, 3-butoxymethyl acrylate, 3-butoxymethyl methacrylate, and 3-butoxymethyl acrylamide; and those comprising a phosphorylcholine group such as 2-methacryloyloxyethyl phosphorylcholine, 2-acryloyloxyethyl phosphorylcholine, 3-(meth)acryloyloxypropyl phosphorylcholine, 4-(meth)acryloyloxybutyl phosphorylcholine, 6-(meth)acryloyloxyhexyl phosphorylcholine, 10-(meth)acryloyloxydecyl phosphorylcholine, co-(meth)acryloyl(poly)oxyethylene phosphorylcholine, 2-acrylamidoethyl phosphorylcholine, 3-acrylamidopropyl phosphorylcholine, 4-acrylamidobutyl phosphorylcholine, 6-acrylamidohexyl phosphorylcholine, 10-acrylamidodecyl phosphorylcholine, and co-(meth)acrylamide(poly)oxyethylene phosphorylcholine.

The hydrophobic monomeric unit is not particularly limited except that the monomeric unit is hydrophobic, but examples thereof include n-butyl acrylate, n-butyl methacrylate, isobutyl acrylate, isobutyl methacrylate, t-butyl acrylate, t-butyl methacrylate, n-hexyl acrylate, n-hexyl methacrylate, n-octyl acrylate, n-octyl methacrylate, n-decyl acrylate, n-decyl methacrylate, n-dodecyl acrylate, n-dodecyl methacrylate, n-tetradecyl acrylate, and n-tetradecyl methacrylate.

In addition, from the viewpoint of suitability of suppressing elution of the hydrophilic polymer, the hydrophilic polymer also preferably comprises a reactive monomeric unit. As the reactive monomeric unit, a UV-reactive monomeric unit is preferable because it is possible to immobilize the hydrophilic polymer on the substrate in a short time treatment, and examples thereof include 4-azidophenyl acrylate, 4-azidophenyl methacrylate, 2-((4-azidobenzoyl)oxy)ethyl acrylate, and 2-((4-azidobenzoyl)oxy)ethyl methacrylate.

The hydrophilic polymer may also contain temperature-responsive monomeric units, and examples thereof include (meth)acrylamide compounds such as acrylamide and methacrylamide; N-alkyl-substituted (meth)acrylamide derivatives such as N,N-diethylacrylamide, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-t-butylacrylamide, N-ethoxyethylacrylamide, N-ethoxyethylmethacrylamide, N-tetrahydrofurfurylacrylamide, and N-tetrahydrofurfurylmethacrylamide; N,N-dialkyl-substituted (meth)acrylamide derivatives such as N,N-dimethyl(meth)acrylamide, N,N-ethylmethylacrylamide, N,N-diethylacrylamide; (meth)acrylamide derivatives comprising cyclic groups such as 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, and 4-(1-oxo-2-methyl-2-propenyl)-morpholine; vinyl ethers such as methyl vinyl ether; and proline derivatives such as N-proline methyl ester acrylamide.

In addition, as another method for preparing the cell culture substrate used in the method for inducing differentiation of the present invention, a method of coating a part of the region on the cell culture substrate with a substance that promotes or inhibits cell adhesiveness by photolithography or inkjet method, a method of coating the surface of the cell culture substrate with a temperature-responsive polymer after subjecting the surface to any one of plasma treatment, ultraviolet treatment, corona discharge treatment, or a combination thereof can be employed.

Moreover, the cell culture substrate used in the method for inducing differentiation of the present invention may be sterilized. Although the method of sterilization is not particularly limited, autoclave sterilization, UV sterilization, γ-ray sterilization, ethylene oxide gas sterilization, and the like can be used. UV sterilization, and ethylene oxide gas sterilization are preferable from the viewpoint of suitability of suppressing deformation of block copolymer, autoclave sterilization, UV sterilization or ethylene oxide gas sterilization are more preferable from the viewpoint of suitability of suppressing deformation of the substrate, and ethylene oxide gas sterilization is particularly preferable from the viewpoint of excellent mass productivity of the cell culture substrate.

In the present invention, the substrate used for the preparation of the cell culture substrate is not particularly limited, but commonly used glass, polystyrene, polycarbonate, polyethylene terephthalate, polyvinylidene fluoride, polyethylene, polypropylene, polyethylene methacrylate and other high molecular compounds, ceramics, and metals can be used. Polystyrene is most preferable from the viewpoint of excellent transparency and easiness of molding and surface modification.

The method for inducing differentiation and production method of the present invention are characterized by performing differentiation induction through the following steps (1-1) to (1-4). (1-1) A step of coating a cell culture substrate with a single or a plurality of substances selected from a group consisting of Matrigel, laminin, fibronectin, vitronectin, collagen, and fragments thereof, and seeding the pluripotent stem cells. (1-2) A step of forming pluripotent stem cell aggregates adhering onto a cell culture substrate by adherently culturing the pluripotent stem cells seeded in the step (1-1) in a feeder-free manner in the presence of an undifferentiated state maintenance medium. (1-3) A step of forming embryoid bodies (embryoids) by culturing the pluripotent stem cell aggregates formed in the step (1-2) in the presence of a medium comprising a differentiation-inducing factor in a state of adhering onto a cell culture substrate. (1-4) A step of forming ectodermal cell aggregates by culturing the embryoid bodies (embryoids) formed in the step (1-3) in the presence of a medium comprising a differentiation-inducing factor in a state of adhering onto the cell culture substrate. The step (1-1) in the method for inducing differentiation of the present invention is a step of coating the cell culture substrate with a single or a plurality of substances selected from the group consisting of Matrigel, laminin, fibronectin, vitronectin, collagen, and fragments thereof, and seeding the pluripotent stem cells. By performing coating with a single or a plurality of substances selected from the group consisting of Matrigel, laminin, vitronectin, fibronectin, collagen, and fragments thereof, the region (A) can be imparted with cell adhesiveness and cell proliferation. When coating is not performed with any of Matrigel, laminin, vitronectin, fibronectin, collagen, and fragments thereof, cell adhesiveness and cell proliferation cannot be imparted. From the viewpoint of suitability of imparting cell adhesiveness and cell proliferation, it is more preferable to use a combination of four types of substances including at least laminin, it is particularly preferable to use a combination of any of laminin and Matrigel, laminin and fibronectin, or laminin and collagen, and it is most preferable to use laminin alone.

The Matrigel, laminin, vitronectin, fibronectin, collagen, and fragments thereof may be natural products, may be artificially synthesized by the genetic recombination technology or the like, or may be fragments cleaved with restriction enzymes or the like, synthetic proteins or synthetic peptides based on these biological materials.

In the present invention, as the Matrigel, for example, Matrigel (manufactured by Corning Incorporated) and Geltrex (manufactured by Gibco) can be suitably used as commercially available products from the viewpoint of their easy availability.

Although the type of laminin is not particularly limited, for example, laminin 511, laminin 521, or laminin 511-E8 fragment, which has been reported to exhibit high activity against α6β1 integrin expressed on the surface of human iPS cells. The laminin may be a natural product, may be one artificially synthesized by the genetic recombination technology or the like, or may be a synthetic protein or synthetic peptide based on the laminin. For example, iMatrix-511 (manufactured by Nippi, Inc.) can be preferably used as a commercial product from the viewpoint of its easy availability.

The vitronectin may be a natural product, may be one artificially synthesized by the genetic recombination technology or the like, or may be a synthetic protein or synthetic peptide based on the vitronectin. For example, Vitronectin, from Human Plasma (manufactured by Wako Pure Chemical Industries, Ltd.), Synthemax (manufactured by Corning Incorporated), and Vitronectin (VTN-N) (manufactured by Gibco) can be preferably used as a commercial product from the viewpoint of their easy availability.

The fibronectin may be a natural product, may be one artificially synthesized by the genetic recombination technology or the like, or may be a synthetic protein or synthetic peptide based on the fibronectin. For example, Fibronectin Solution, from Human Plasma (manufactured by Wako Pure Chemical Industries, Ltd.), and Retronectin (manufactured by Takara Bio Inc.) can be preferably used as a commercial product from the viewpoint of their easy availability.

Although the type of collagen is not particularly limited, for example, type I collagen or type IV collagen can be used. The collagen may be a natural product, may be one artificially synthesized by the genetic recombination technology or the like, or may be a synthetic peptide based on the collagen. For example, Collagen I, Human (manufactured by Corning Incorporated), and Collagen IV, Human (manufactured by Corning Incorporated) can be preferably used as a commercial product from the viewpoint of their easy availability.

The type of cells in the step (1-1) can be appropriately selected from pluripotent stem cells such as ES cells and iPS cells, but iPS cells are preferable from the viewpoint of suitability of applying the method for inducing differentiation of the present invention for regenerative medicine and drug discovery. The cell seeding method in the step (1-1) is not particularly limited, but examples thereof include a method of adding a cell suspension in which cells are monodispersed to the cell culture substrate, or adding cell aggregates to the cell culture substrate, and a method of monodispersing and seeding is preferable from the viewpoint that the cell aggregates are uniformly formed in the region (A). When human-derived iPS cells are monodispersed and cultured, a Rhobinding kinase (ROCK) inhibitor is preferably added at the time of seeding in order to suppress apoptosis due to low cell density. Examples of ROCK inhibitors include (R)-(+)-trans-N-(4-pyridyl)-4-(1-aminoethyl)-cyclohexanecarboxamide 2HCl H₂O (manufactured by Fujifilm Wako Pure Chemical Corporation, product name: Y-27632) and the like. The concentration of the ROCK inhibitor added to the medium is in a range effective for maintaining the survival of human cells and does not affect the undifferentiated state of human cells, preferably 1 µM to 50 µM, more preferably 3 µM to 20 µM, still more preferably 5 µM to 15 µM, most preferably 8 µM to 12 µM. Moreover, the ROCK inhibitor is preferably removed 24 hours after seeding the cells by a method such as replacing the medium with a medium that does not contain the ROCK inhibitor.

Although the method for collecting cells is not particularly limited, for example, enzymatic treatment with trypsin or collagenase, chelate treatment with ethylenediamineacetic acid (EDTA), or physical treatment with a scraper or the like can be used singly or in combination. When enzymatic treatment is used, it is preferable to be free of animal-derived components. TrypLE Select (manufactured by Gibco), TrypLE Express (Gibco), Accutase (manufactured by Nacalai Tesque Inc.), and Accumax (manufactured by Nacalai Tesque Inc.) can be preferably used as a commercial product from the viewpoint of their easy availability.

From the viewpoint of suitability of forming uniform cell aggregates in a short period of time, the cell seeding density in the step (1-1) is preferably 1.0 × 10² cells/cm² or more, more preferably 1.2 × 10³ cells/cm² or more, particularly preferably 2.0 × 10³ cells/cm² or more, and most preferably 3.0 × 10³ cells/cm² or more. In addition, from the viewpoint of suitability of suppressing cell death due to insufficient nutrition in the medium, the cell seeding density is preferably 1.0 × 10⁵ cells/cm² or less, more preferably 5.0 × 10⁴ cells/cm² or less, particularly preferably 2.5 × 10⁴ cells/cm² or less, and most preferably 5.0 × 10³ cells/cm² or less.

In the step (1-2) in the method for inducing differentiation of the present invention, the pluripotent stem cell aggregates adhering onto the cell culture substrate are formed by adherently culturing the pluripotent stem cells seeded in the step (1-1) in a feeder-free manner in the presence of an undifferentiated state maintenance medium. In the present invention, the term "feeder-free" refers to a method of seeding and culturing the pluripotent stem cells directly on the cell culture substrate without using cells (feeder cells) that have been inactivated by gamma irradiation or the like. By adherently culturing the pluripotent stem cells seeded in the step (1-1) in a feeder-free manner in the presence of an undifferentiated state maintenance medium, the pluripotent stem cells can proliferate in the region (A), and the cell aggregates layered in the out-of-plane direction of the substrate can be formed. When the undifferentiated state maintenance medium is not used, cell aggregates lose differentiation pluripotency, and the differentiation induction efficiency into ectodermal cells deteriorates. In addition, by culturing the pluripotent stem cells in a feeder-free manner, it is possible to facilitate maintenance of nutrients in the medium during culture, and enhance cell viability and maintenance of undifferentiated cells. In addition, by the adherent culture, it is easier to exchange the medium than the suspension culture, and thus culture workability is excellent, and it is possible to exchange the entire amount of the medium. Therefore, the concentration of waste products discharged from the cells can be kept low. In addition, since the influence of the concentration gradient in the culture system is low, the differentiation induction efficiency is excellent.

As the undifferentiated state maintenance medium, examples of factors acting to maintain the undifferentiated state of pluripotent stem cells include basic fibroblast growth factor (bFGF), transforming growth factor β (TGF-β), insulin-like growth factor (IGF), activin A, Wnt, insulin, transferrin, ethanolamine, 2-mercaptoethanol, selenic acid, oleic acid, and sodium bicarbonate, a factor comprising at least one of these is preferable, and a factor comprising bFGF, which acts to maintain a highly undifferentiated state is most preferable. Although the type of medium is not particularly limited, examples thereof include commercially available undifferentiated state maintenance mediums, such as a culture medium obtained by adding a factor acting to maintain an undifferentiated state and culture supplements such as non-essential amino acids to a basal medium such as DMEM, Ham's F12, and D-MEM/Ham's F12, Primate ES Cell Medium (manufactured by REPROCELL Inc.), StemFit AK02N (manufactured by Ajinomoto Co., Inc.), StemFit AK03 (manufactured by Ajinomoto Co., Inc.), mTeSR1 (manufactured by STEMCELL Technologies), TeSR-E8 (manufactured by STEMCELL Technologies), ReproNaive (manufactured by REPROCELL Inc.), ReproXF (manufactured by REPROCELL Inc.), ReproFF (manufactured by REPRO CELL Inc.), ReproFF2 (manufactured by REPRO CELL Inc.), NutriStem (manufactured by Biological Industries), iSTEM (manufactured by Takara Bio Inc.), GS2-M (manufactured by Takara Bio Inc.), and hPSC Growth Medium DXF (manufactured by PromoCell Inc.). From the viewpoint of suitability of stably maintaining the undifferentiated state of cells, Primate ES Cell Medium, StemFit AK02N, and StemFit AK03 are preferable, StemFit AK02N and StemFit AK03 are more preferable, and StemFit AK02N (manufactured by Ajinomoto Co., Inc.) is most preferable. Moreover, since serum which is generally used in cell culture mediums also comprises differentiation-inducing factors, the undifferentiated state maintenance medium for pluripotent stem cells is preferably a serum-free medium.

In addition, in the step (1-2), a ROCK inhibitor similar to the step (1-1) may be added in order to enhance the survival rate of pluripotent stem cells. The types and concentrations of suitable ROCK inhibitors are the same as described above.

In addition, in the step (1-2), from the viewpoint of suitability of enhancing the differentiation induction efficiency in the steps (1-3) and (1-4) described later, it is preferable to perform culturing until the number of cells per unit area of the region (A) reaches 1.0 × 10⁴ cells/cm² or more, more preferably 2.0 × 10⁴ cells/cm² or more, particularly preferably 5.0 × 10⁴ cells/cm² or more, and most preferably 7.5 × 10⁴ cells/cm² or more. In addition, from the viewpoint of suitability of maintaining nutrients in the medium and enhancing the survival rate of cells in the steps (1-3) and (1-4), it is preferable to proceed to the step (1-3) when the number of cells per unit area of the region (A) is 5.0 × 10⁶ cells/cm² or less, more preferably 1.0 × 10⁶ cells/cm² or less, particularly preferably 5.0 × 10⁵ cells/cm² or less, and most preferably 1.0 × 10⁵ cells/cm² or less. In order to obtain the number of cells per unit area of the region (A), it is preferable to perform culturing in the step (1-2) for 1 to 48 hours, more preferably 6 to 36 hours, particularly preferably 12 to 36 hours, and most preferably 18 to 30 hours.

In addition, from the viewpoint of suitability of enhancing the differentiation induction efficiency in the steps (1-3) and (1-4) described later, the shape of the embryoid bodies (embryoids) formed in the step (1-2) is preferably a hemispherical shape. In the step (1-3) in the method for inducing differentiation of the present invention, the pluripotent stem cell aggregates formed in the step (1-2) are cultured in the presence of a medium comprising a differentiation-inducing factor in a state of adhering onto a cell culture substrate to form embryoid bodies (embryoids).

From the viewpoint of suitability of enhancing the differentiation induction efficiency of embryoid bodies (embryoids), the differentiation-inducing factor in the step (1-3) preferably comprises an ectoderm-inducing factor, a mesoderm-inducing factor, and an endoderm-inducing factor. As the ectoderm-inducing factors, from the viewpoint of suitability of enhancing the differentiation induction efficiency of embryoid bodies (embryoids), it is preferable to contain any one of Noggin (BMP inhibitor), dorsomorphin (BMP inhibitor), SB431542 (TGF-β inhibitor), and an activin inhibitor, and more preferable to contain a BMP inhibitor and a TGF-β inhibitor. In addition, as the mesoderm-inducing factor, from the viewpoint of enhancing the differentiation induction efficiency of embryoid bodies (embryoids), it is preferable to contain any one of activin A, CHIR99021 (GSK3β inhibitor), and bone morphogenetic protein (BMP) 4, and more preferable to contain a GSK3β inhibitor. Further, the endoderm-inducing factors include low-molecular-weight compounds such as activin A and CHIR99021.

In addition, the concentration of the differentiation-inducing factor added to the medium is not particularly limited, and from the viewpoint of enhancing the differentiation induction efficiency of embryoid bodies (embryoids), the concentration is preferably 0.1 to 10 µM, more preferably 1.0 to 7.5 µM, and most preferably 2.0 to 5.0 µM. The medium comprising the differentiation-inducing factor is preferably exchanged every 24 hours during the culture such that the differentiation induction proceeds sufficiently. By exchanging the medium within 24 hours, it is possible to prevent a shortage of differentiation-inducing factors in the medium and to uniformly differentiate all the cells.

As ectoderm markers possessed by the embryoid bodies (embryoids) formed in the step (1-3), PAX6, Nestin, SOX1, SOX2, SOX10, Notch1, E Cadherin, and MAP2 are preferable, and PAX6, Nestin, SOX1, and SOX2 are more preferable. In addition, as mesoderm markers, Tbx1, Brachyury, MSX1, and Flk-1 are preferable, and Tbx1 and Brachyury are more preferable. Further, as endoderm markers, FOXA2, SOX17, GATA4, GATA6, CXCR4, HNF3β, HNF4α, and αFP are preferable, and FOXA2 and SOX17 are more preferable. In addition, as neural stem cell markers, PAX6, Nestin, Olig2, SOX1, SOX2, and DCX are preferable, and PAX6, Nestin, SOX1, and Olig2 are more preferable.

In the step (1-4) in the method for inducing differentiation of the present invention, the embryoid bodies (embryoids) formed in the step (1-3) are cultured in the presence of a medium comprising a differentiation-inducing factor in a state of adhering onto a cell culture substrate to form ectodermal cell aggregates. By inducing the differentiation of the embryoid bodies (embryoids) formed in the step (1-3) in a state of adhering onto the cell culture substrate, the concentration gradient of the above-described inducing factor has little effect, and thus the uniform differentiation is expected to proceed. The medium is not particularly limited, but examples thereof include a culture medium obtained by adding a factor acting ectoderm induction and a culture supplement to a basal medium such as DMEM, Ham's F12, and D-MEM/Ham's F12.

The types of cells induced to differentiate from the embryoid bodies (embryoids) in the step (1-4) are not particularly limited except that the cells are ectodermal cells, but for example, epidermal ectodermal-derived cells and neuroectodermal-derived cells are preferable, and neuroectodermal-derived cells are more preferable from the viewpoint of using for regenerative medicine and drug discovery. Neuroectodermal-derived cells are not particularly limited, but for example, Schwann progenitor cells, myelinating Schwann cells, non-myelinating Schwann cells, radial glial cells, oligodendrocyte progenitor cells, neuroglial cells such as oligodendrocytes and astrocytes, glutamatergic neurons, GABAergic neurons, dopaminergic nerve cells, serotonergic nerve cells, cholinergic nerve cells, motor nerve cells, sensory nerve cells, and the like are preferable. When the ectodermal-derived cells are nerve cells, it is preferable to culture for 3 to 5 days in the presence of a medium comprising B27 supplement, TGF-β inhibitor, GSK3β inhibitor, LIF, bFGF, retinoic acid, purmorphamine, and DAPT, after performing culturing for 5 to 14 days in the presence of a medium comprising B27 supplement, TGF-β inhibitor, GSK3β inhibitor, LIF, bFGF, retinoic acid, and purmorphamine. When further differentiating into motor nerve cells, it is preferable to culture the nerve cells in the presence of a medium comprising B27 supplement, rhBDNF, rhGDNF, ascorbic acid, retinoic acid, and DAPT for 5 to 40 days after culturing the above-described nerve cells.

### <Method for inducing differentiation into mesodermal cells>

Hereinafter, the mode for carrying out the method for inducing differentiation into mesodermal cells according to the present invention will be described in detail.

The method for inducing differentiation of the present invention uses a cell culture substrate having the following two regions (A) and (B). (A) An island-shaped region with an area of 0.001 to 5 mm² having cell adhesiveness and cell proliferation. (B) A region adjacent to the region (A) and having no cell adhesiveness or having no cell proliferation.

By using the cell culture substrate having the region (A) as the cell culture substrate, pluripotent stem cells can be adherently cultured on the cell culture substrate in the step (2-2) described later, and the embryoid bodies (embryoids) adhering to the cell culture substrate can be formed. When there is no region (A), pluripotent stem cells cannot be adherently cultured on the cell culture substrate and embryoid bodies (embryoids) adhering to the cell culture substrate cannot be formed. In addition, by using a cell culture substrate having the region (B) as the cell culture substrate, pluripotent stem cells can proliferate only in the region (A), and uniform-sized embryoid bodies (embryoids) can be formed. Differentiation induction efficiency can be enhanced by forming uniform-sized embryoid bodies (embryoids). When there is no region (B), uniform-sized embryoid bodies (embryoids) cannot be formed.

As the area of the region (A), from the viewpoint of suitability of forming embryoid bodies (embryoids) having a size suitable for inducing differentiation into mesodermal cells, the area of the region (A) is more preferably 0.005 to 3 mm², particularly preferably 0.01 to 1.0 mm², and most preferably 0.03 to 0.8 mm².

The shape of the region (A) is the same as in <Method for inducing differentiation into ectodermal cells>.

The shape of the region (B) is the same as in <Method for inducing differentiation into ectodermal cells>.

The area ratio of the region (A) and the region (B) is the same as in <Method for inducing differentiation into ectodermal cells>.

The cell culture substrate used in the method for inducing differentiation of the present invention forms uniform-sized embryoid bodies (embryoids), and from the viewpoint of suitability of enhancing differentiation induction efficiency, it is preferable that the layer comprising hydrophilic polymer be contained on the surface, and the region (A) be a region in which a layer comprising the hydrophilic polymer is decomposed or modified by plasma treatment, ultraviolet treatment, and/or corona discharge treatment. By comprising a layer comprising hydrophilic polymer on the surface of the cell culture substrate, adsorption of proteins that contribute to substrate-cell adhesiveness in the region (B) is suppressed, and the region (B) can be set to a region having no cell adhesiveness or having no cell proliferation. In addition, by partially decomposing or modifying the layer comprising the hydrophilic polymer, the region (A) can be imparted with cell adhesiveness or cell proliferation. In addition, from the viewpoint of the suitability of enhancing the cell adhesiveness and cell proliferation of the region (A) and forming embryoid bodies (embryoids) in a short period of time, it is more preferable that the region (A) be a plasma-treated region.

In addition, in the cell culture substrate used in the method for inducing differentiation of the present invention, from the suitability of enhancing cell proliferation, it is preferable that the region (A) be a region without a hydrophilic polymer and the region (B) be a region comprising a layer comprising hydrophilic polymer, and it is more preferable that the region (A) be a region in which the substrate surface modified by plasma treatment, ultraviolet treatment and/or corona discharge treatment is exposed, the region (A) be a region that does not comprising hydrophilic polymer and the region (B) be a region comprising a layer comprising hydrophilic polymer.

The layer thickness of the layer comprising the hydrophilic polymer is the same as in <Method for inducing differentiation into ectodermal cells>.

The method for forming the layer comprising the hydrophilic polymer is the same as in <Method for inducing differentiation into ectodermal cells>.

The type of the hydrophilic polymer is the same as in <Method for inducing differentiation into ectodermal cells>.

Regarding the hydrophilic polymer, from the viewpoint of suitability of suppressing the elution of the hydrophilic polymer from the cell culture substrate, and suppressing the influence on the quality due to the contamination of the cell aggregates and the embryoid bodies (embryoids) by the polymer, a random copolymer or a block copolymer having both hydrophilic monomeric units and hydrophobic monomeric units is preferable, and a random copolymer having both hydrophilic monomeric units and hydrophobic monomeric units is more preferable. In addition, the composition ratio of the copolymer is the same as in <Method for inducing differentiation into ectodermal cells>.

The hydrophilic monomeric unit is the same as in <Method for inducing differentiation into ectodermal cells>.

The hydrophobic monomeric unit is the same as in <Method for inducing differentiation into ectodermal cells>.

In addition, from the viewpoint of suitability of suppressing elution of the hydrophilic polymer, the hydrophilic polymer also preferably comprises a reactive monomeric unit. The reactive monomeric unit is the same as in <Method for inducing differentiation into ectodermal cells>.

The hydrophilic polymer may also contain temperature-responsive monomeric units, and the temperature-responsive monomeric unit is the same as in <Method for inducing differentiation into ectodermal cells>.

In addition, another method for preparing a cell culture substrate used in the method for inducing differentiation of the present invention is the same as in <Method for inducing differentiation into ectodermal cells>.

Moreover, the cell culture substrate used in the method for inducing differentiation of the present invention may be sterilized. The sterilization method is the same as in <Method for inducing differentiation into ectodermal cells>.

In the present invention, the substrate used for preparing the cell culture substrate is the same as in <Method for inducing differentiation into ectodermal cells>.

The method for inducing differentiation of the present invention is characterized by performing differentiation induction through the following steps (2-1) to (2-3). (2-1) A step of coating a cell culture substrate with a single or a plurality of substances selected from a group consisting of Matrigel, laminin, fibronectin, vitronectin, collagen, and fragments thereof, and seeding the pluripotent stem cells. (2-2) A step of forming embryoid bodies (embryoids) adhering onto a cell culture substrate by adherently culturing the pluripotent stem cells seeded in the step (2-1) in a feeder-free manner in the presence of an undifferentiated state maintenance medium. (2-3) A step of forming mesodermal cell aggregates by culturing the embryoid bodies (embryoids) formed in the step (2-2) in the presence of a medium comprising a differentiation-inducing factor in a state of adhering onto the cell culture substrate.

The step (2-1) in the method for inducing differentiation of the present invention is a step of coating the cell culture substrate with a single or a plurality of substances selected from the group consisting of Matrigel, laminin, fibronectin, vitronectin, collagen, and fragments thereof, and seeding the pluripotent stem cells.

By performing coating with a single or a plurality of substances selected from the group consisting of Matrigel, laminin, fibronectin, vitronectin, collagen, and fragments thereof, the region (A) can be imparted with cell adhesiveness and cell proliferation. When coating is not performed with any of Matrigel, laminin, fibronectin, vitronectin, collagen, and fragments thereof, cell adhesiveness and cell proliferation cannot be imparted. From the viewpoint of suitability of imparting cell adhesiveness and cell proliferation, it is more preferable to use a combination of four types of substances including at least laminin, it is particularly preferable to use a combination of any of laminin and Matrigel, laminin and fibronectin, or laminin and collagen, and it is most preferable to use laminin alone.

The Matrigel, laminin, fibronectin, vitronectin, collagen, and fragments thereof are the same as in <Method for inducing differentiation into ectodermal cells>.

The type of cells in the step (2-1) can be appropriately selected from pluripotent stem cells such as ES cells and iPS cells, but iPS cells are preferable from the viewpoint of suitability of applying the method for inducing differentiation of the present invention for regenerative medicine and drug discovery. The cell seeding method in the step (2-1) is not particularly limited, but examples thereof include a method of adding a cell suspension in which cells are monodispersed to the cell culture substrate, or adding cell aggregates to the cell culture substrate, and a method of monodispersing and seeding is preferable from the viewpoint that the cell aggregates are uniformly formed in the region (A). When human-derived iPS cells are monodispersed and cultured, a Rho-binding kinase (ROCK) inhibitor is preferably added at the time of seeding in order to suppress apoptosis due to low cell density. The ROCK inhibitor is the same as in <Method for inducing differentiation into ectodermal cells>.

From the viewpoint of suitability of forming uniform cell aggregates in a short period of time, the cell seeding density in the step (2-1) is preferably 1.0 × 10² cells/cm² or more, more preferably 1.2 × 10³ cells/cm² or more, particularly preferably 2.0 × 10³ cells/cm² or more, and most preferably 3.0 × 10³ cells/cm² or more. In addition, from the viewpoint of suitability of suppressing cell death due to insufficient nutrition in the medium, the cell seeding density is preferably 1.0 × 10⁶ cells/cm² or less, more preferably 5.0 × 10⁵ cells/cm² or less, particularly preferably 2.5 × 10⁴ cells/cm² or less, and most preferably 5.0 × 10³ cells/cm² or less.

In the step (2-2) in the method for inducing differentiation of the present invention, the embryoid bodies (embryoids) adhering onto the cell culture substrate are formed by adherently culturing the pluripotent stem cells seeded in the step (2-1) in a feeder-free manner in the presence of an undifferentiated state maintenance medium. In the present invention, the term "feeder-free" refers to a method of seeding and culturing the pluripotent stem cells directly on the cell culture substrate without using cells (feeder cells) that have been inactivated by gamma irradiation or the like. By adherently culturing the pluripotent stem cells seeded in the step (2-1) in a feeder-free manner in the presence of an undifferentiated state maintenance medium, the pluripotent stem cells can proliferate in the region (A), and the embryoid bodies (embryoids) layered in the out-of-plane direction of the substrate can be formed. When the undifferentiated state maintenance medium is not used, embryoid bodies (embryoids) lose differentiation pluripotency, and the differentiation induction efficiency into mesodermal cells deteriorates. In addition, by culturing the pluripotent stem cells in a feeder-free manner, it is possible to facilitate maintenance of nutrients in the medium during culture, and enhance cell viability and maintenance of undifferentiated cells. In addition, by the adherent culture, it is easier to exchange the medium than the suspension culture, and thus culture workability is excellent, and it is possible to exchange the entire amount of the medium. Therefore, the concentration of waste products discharged from the cells can be kept low. In addition, since the influence of the concentration gradient in the culture system is low, the differentiation induction efficiency is excellent.

The undifferentiated state maintenance medium is the same as in <Method for inducing differentiation into ectodermal cells>.

In addition, in the step (2-2), the ROCK inhibitor similar to the step (2-1) may be added in order to enhance the survival rate of pluripotent stem cells. The types and concentrations of suitable ROCK inhibitors are the same as described above.

In addition, in the step (2-2), from the viewpoint of suitability of enhancing the differentiation induction efficiency in the step (2-3) described later, it is preferable to perform culturing until the number of cells per unit area of the region (A) reaches 1.0 × 10⁴ cells/cm² or more, more preferably 2.0 × 10⁴ cells/cm² or more, particularly preferably 5.0 × 10⁴ cells/cm² or more, and most preferably 7.5 × 10⁴ cells/cm² or more. In addition, from the viewpoint of suitability of maintaining nutrients in the medium and enhancing the survival rate of cells in the step (2-3), it is preferable to proceed to the step (2-3) when the number of cells per unit area of the region (A) is 5.0 × 10⁶ cells/cm² or less, more preferably 1.0 × 10⁶ cells/cm² or less, particularly preferably 5.0 × 10⁵ cells/cm² or less, and most preferably 1.0 × 10⁵ cells/cm² or less.

In order to obtain the number of cells per unit area of the region (A), it is preferable to perform culturing in the step (2-2) for 1 to 48 hours, more preferably 6 to 36 hours, particularly preferably 12 to 36 hours, and most preferably 18 to 30 hours.

In addition, since it is suitable for enhancing the differentiation induction efficiency in the step (2-3) described later, the shape of the embryoid bodies (embryoids) formed in the step (2-2) can be set as appropriate, and examples thereof include a circle, an ellipse, a polygon, and a closed shape composed of an irregular straight line or a curved line. In addition, from the viewpoint of suitability of producing spheroids having a shape close to a sphere, the island shape is preferably circular, elliptical, or polygonal, more preferably circular, elliptical, or rectangular, particularly preferably circular, elliptical, or square, and most preferably circular or elliptical.

In addition, in the present invention, from the viewpoint of suitability of producing spheroids having a shape close to a sphere, the aspect ratio of the island shape is preferably 5 or less, more preferably 2 or less, particularly preferably 1.5 or less, and most preferably 1.1 or less. Here, the "aspect ratio" in the present invention indicates major diameter/minor diameter, which is the ratio of the maximum diameter (major diameter) to the minimum diameter (minor diameter) of the shape.

In the step (2-3) in the method for inducing differentiation of the present invention, the embryoid bodies (embryoids) formed in the step (2-2) are cultured in the presence of a medium comprising a differentiation-inducing factor in a state of adhering onto a cell culture substrate to form mesodermal cells.

From the viewpoint of suitability of enhancing the differentiation induction efficiency into mesodermal cells, the differentiation-inducing factor in the step (2-3) preferably comprises a mesoderm-inducing factor. As the mesoderm-inducing factor, from the viewpoint of enhancing the differentiation induction efficiency of embryoid bodies (embryoids), single or a plurality of differentiation-inducing factors selected from the group consisting of a GSK3β inhibitor, bone morphogenetic protein (BMP), and activin are preferable, and it is particularly preferable to contain any one of activin A, CHIR99021 (GSK3β inhibitor), and BMP4.

In addition, the concentration of the differentiation-inducing factor added to the medium is not particularly limited, and from the viewpoint of enhancing the differentiation induction efficiency of embryoid bodies (embryoids), the concentration is preferably 1000 to 500 ng/mL, more preferably 500 to 100 ng/mL, and most preferably 100 ng/mL or less.

The medium comprising the differentiation-inducing factor is preferably exchanged every 24 hours during the culture such that the differentiation induction proceeds sufficiently. By exchanging the medium within 24 hours, it is possible to prevent a shortage of differentiation-inducing factors in the medium and to uniformly differentiate all the cells.

Mesoderm markers possessed by the embryoid bodies (embryoids) formed in the step (2-2) include FLK-1, MESP1, MESP2, FOXF1, HAND1, EVX1, IRX3, CDX2, TBX6, MIXL1, SNAI1, FOXC1, and PDGFRα are preferable, and BRACHYURY is most preferable.

In the step (2-3) in the method for inducing differentiation of the present invention, the embryoid bodies (embryoids) formed in the step (2-2) are cultured in the presence of a medium comprising a differentiation-inducing factor in a state of adhering onto a cell culture substrate to form mesodermal cell aggregates. By inducing the differentiation of the embryoid bodies (embryoids) formed in the step (2-2) in a state of adhering onto the cell culture substrate, the concentration gradient of the above-described inducing factor has little effect, and thus the uniform differentiation is expected to proceed. The medium used in the step (2-3) is not particularly limited, but examples thereof include a culture medium obtained by adding the mesoderm-inducing factor and a culture supplement to a basal medium such as DMEM, Ham's F12, and D-MEM/Ham's F12.

The types of cells induced to differentiate from the embryoid bodies (embryoids) in the step (2-3) are not particularly limited except that the cells are mesodermal cells, but for example, blood cells, smooth muscle cells, and germ cells are preferable, and osteocytes, cardiomyocytes, skeletal muscle cells, and renal cells are more preferable from the viewpoint of using for regenerative medicine and drug discovery.

### <Method for inducing differentiation into endodermal cells>

Hereinafter, the mode for carrying out the method for inducing differentiation into endodermal cells according to the present invention will be described in detail.

The method for inducing differentiation of the present invention uses a cell culture substrate having the following region (A) and region (B).
(A) An island-shaped region with an area of 0.001 to 5 mm² having cell adhesiveness and cell proliferation.
(B) A region adjacent to the region (A) and having no cell adhesiveness or having no cell proliferation.

By using the cell culture substrate having the region (A) as the cell culture substrate, pluripotent stem cells can be adherently cultured on the cell culture substrate in the step (3-2) described later, and the embryoids adhering to the cell culture substrate can be formed. When there is no region (A) in the cell culture substrate, pluripotent stem cells cannot be adherently cultured on the cell culture substrate, and embryoids adhering to the cell culture substrate cannot be formed. In addition, by using a cell culture substrate having the region (B) as the cell culture substrate, pluripotent stem cells can proliferate only in the region (A), and uniform-sized embryoids can be formed. Differentiation induction efficiency can be enhanced by forming uniform-sized embryoids. When there is no region (B) in the cell culture substrate, uniform-sized embryoids cannot be formed.

As the area of the region (A), from the viewpoint of suitability of forming embryoids having a size suitable for inducing differentiation into endodermal cells, the area of the region (A) is more preferably 0.005 to 3 mm², still more preferably 0.02 to 2.5 mm², and most preferably 0.03 to 2.0 mm².

The shape of the region (A) is the same as in <Method for inducing differentiation into ectodermal cells>.

The cell culture substrate used in the method for inducing differentiation of the present invention forms uniform-sized embryoids, and from the viewpoint of suitability of enhancing differentiation induction efficiency, it is preferable that the layer comprising hydrophilic polymer be contained on the surface, and the region (A) be a region in which a layer comprising the hydrophilic polymer is decomposed or modified by at least one treatment selected from the group consisting of plasma treatment, ultraviolet treatment, and corona discharge treatment. By comprising a layer comprising hydrophilic polymer on the surface of the cell culture substrate, a region in which adsorption of proteins that contribute to substrate-cell adhesiveness in the region (B) is suppressed, and the region (B) can be set to a region having no cell adhesiveness or having no cell proliferation. In addition, by partially decomposing or modifying the layer comprising the hydrophilic polymer, the region (A) can be imparted with cell adhesiveness or cell proliferation. In addition, from the viewpoint of the suitability of enhancing the cell adhesiveness and cell proliferation of the region (A) and forming embryoids in a short period of time, it is more preferable that the region (A) be a region decomposed and modified by plasma treatment.

In addition, in the cell culture substrate used in the method for inducing differentiation of the present invention, from the viewpoint of suitability of enhancing cell proliferation, it is preferable that the region (A) be a region without a hydrophilic polymer and the region (B) be a region comprising a layer comprising hydrophilic polymer, and it is more preferable that the region (A) be a region in which the substrate surface modified by plasma treatment, ultraviolet treatment and/or corona discharge treatment is exposed and the region (B) be a region comprising a layer comprising hydrophilic polymer.

The layer thickness of the layer comprising the hydrophilic polymer is the same as in <Method for inducing differentiation into ectodermal cells>.

Examples of the method for forming the layer comprising the hydrophilic polymer include the same methods as the forming method exemplified for the method for inducing differentiation into ectodermal cells.

Examples of the type of the hydrophilic polymer include the same hydrophilic polymers as those exemplified for the method for inducing differentiation into ectodermal cells.

Regarding the hydrophilic polymer, from the viewpoint of suitability of suppressing the elution of the hydrophilic polymer from the cell culture substrate, and suppressing the influence on the quality due to the contamination of the cell aggregates and the embryoids by the polymer, a random copolymer or a block copolymer having both hydrophilic monomeric units and hydrophobic monomeric units is preferable, and a random copolymer having both hydrophilic monomeric units and hydrophobic monomeric units is more preferable. In addition, the composition ratio of the copolymer is the same as in <Method for inducing differentiation into ectodermal cells>.

The hydrophilic monomeric unit is the same as in <Method for inducing differentiation into ectodermal cells>.

The hydrophobic monomeric unit is the same as in <Method for inducing differentiation into ectodermal cells>.

From the viewpoint of suitability of suppressing elution of the hydrophilic polymer, the hydrophilic polymer also preferably comprises a reactive monomeric unit. The reactive monomeric unit is the same as in <Method for inducing differentiation into ectodermal cells>.

The hydrophilic polymer may also contain temperature-responsive monomeric units. The temperature-responsive monomeric unit is the same as in <Method for inducing differentiation into ectodermal cells>.

In addition, another method for preparing a cell culture substrate used in the method for inducing differentiation of the present invention is the same as in <Method for inducing differentiation into ectodermal cells>.

The region (B) is adjacent to the region (A) and has no cell adhesiveness or having no cell proliferation. Since the region (B) is adjacent to the region (A) and does not have cell proliferation, when the cells are cultured, it is possible to form cell aggregates only in the region (A), and to form a state where no cells exist around the region (A). In addition, from the viewpoint of suitability of uniformizing the size and shape of aggregates to be produced, the region (B) preferably has neither cell proliferation nor cell adhesiveness.

The shape of the region (B) is the same as in <Method for inducing differentiation into ectodermal cells>.

The area ratio of the region (A) and the region (B) is the same as in <Method for inducing differentiation into ectodermal cells>.

Moreover, the cell culture substrate used in the method for inducing differentiation of the present invention may be sterilized. The sterilization method is the same as in <Method for inducing differentiation into ectodermal cells>.

In the present invention, the substrate used for preparing the cell culture substrate is the same as in <Method for inducing differentiation into ectodermal cells>.

The method for inducing differentiation of the present invention includes the following steps (3-1), (3-2), and (3-3).

Step (3-1): A step of adding a composition comprising at least one selected from laminin and fragments thereof to a cell culture substrate having the above-described regions (A) and (B) based on an area of a culture surface of the cell culture substrate such that a total amount of laminin and fragments thereof is 1 to 100 µg/cm², and seeding the pluripotent stem cells.

Step (3-2): A step of forming embryoids adhering onto the culture surface of the cell culture substrate by adherently culturing the pluripotent stem cells seeded in the step (3-1) in a feeder-free manner in the presence of an undifferentiated state maintenance medium.

Step (3-3): A step of forming endodermal cell aggregates by culturing the embryoids formed in the step (3-2) in the presence of a medium comprising a differentiation-inducing factor in a state of adhering onto the culture surface of the cell culture substrate, and by performing differentiation induction.

The step (3-1) is a step of adding a composition comprising at least one selected from laminin and fragments thereof to the above-described cell culture substrate based on an area of a culture surface of the cell culture substrate such that a total amount of laminin and fragments thereof is 1 to 100 µg/cm², and seeding the pluripotent stem cells. In addition, the culture surface of the cell culture substrate is the surface to which cells can adhere among the surfaces that come into contact with the medium during culture (a surface which is usually perpendicular to the vertical direction since cells settle due to gravity). By adding a composition comprising at least one selected from laminin and fragments thereof, the culture surface of the cell culture substrate is coated with at least one selected from laminin and fragments thereof. As a result, the cell adhesiveness and cell proliferation of the region (A) are maintained during culture during differentiation induction. The cell adhesiveness and cell proliferation cannot be maintained unless coated with at least one selected from laminin and fragments thereof. From the viewpoint of suitability of maintaining the cell adhesiveness during culture during differentiation induction, the composition comprising at least one selected from laminin and fragments thereof is more preferably a composition comprising at least one selected from laminin and fragments thereof. Regarding the amount of the composition comprising at least one selected from laminin and fragments thereof to be added, based on the area of the culture surface of the cell culture substrate, the total amount of laminin and fragments thereof is more preferably an amount of 1.2 to 50 µg/cm², and most preferably an amount of 1.5 to 10 µg/cm². When the amount added is less than 1 µg/cm², cell adhesiveness cannot be maintained during culture during differentiation induction.

When coating the culture surface of the cell culture substrate with at least one selected from laminin and fragments thereof, a precoating method may be used in which a solution (composition) in which at least one selected from laminin and fragments thereof is diluted with PBS or the like is added to the cell culture substrate and allowed to stand for several hours, or an addition method may be used in which a composition comprising at least one selected from laminin and fragments thereof is added to a cell culture substrate in a state of being mixed with a cell suspension when seeding pluripotent stem cells. That is, in the step (3-1), the addition of a composition comprising at least one selected from laminin and fragments thereof may be performed before seeding pluripotent stem cells, or may be performed at the same time.

Laminin and fragments thereof are the same as in <Method for inducing differentiation into ectodermal cells>.

The type of pluripotent stem cells seeded in the step (3-1) can be appropriately selected from pluripotent stem cells such as ES cells and iPS cells, but iPS cells are preferable from the viewpoint of suitability of applying the method for regenerative medicine and drug discovery. The method of seeding pluripotent stem cells in step (3-1) is not particularly limited, and examples thereof include a method in which cell suspension in which pluripotent stem cells are monodispersed is added to the cell culture substrate, or in which cell aggregates are added to the cell culture substrate. Among these methods, the cell aggregates are uniformly formed in the region (A), and thus the cells are preferably monodispersed and seeded. When human-derived iPS cells are monodispersed and cultured, a Rho-binding kinase (ROCK) inhibitor is preferably added at the time of seeding in order to suppress apoptosis due to low cell density. The ROCK inhibitor is the same as in <Method for inducing differentiation into ectodermal cells>.

From the viewpoint of suitability of forming uniform cell aggregates in a short period of time, the cell seeding density in the step (3-1) is preferably 1.0 × 10² cells/cm² or more, more preferably 1.2 × 10³ cells/cm² or more, still more preferably 2.0 × 10³ cells/cm² or more, and most preferably 3.0 × 10³ cells/cm² or more. In addition, from the viewpoint of suitability of suppressing cell death due to insufficient nutrition in the medium, the cell seeding density is preferably 1.0 × 10⁶ cells/cm² or less, more preferably 5.0 × 10⁵ cells/cm² or less, still more preferably 1.0 × 10⁵ cells/cm² or less, and most preferably 2.5 × 10⁴ cells/cm² or less.

The step (3-2) is a step of forming the embryoids adhering onto the culture surface of the cell culture substrate by adherently culturing the pluripotent stem cells seeded in the step (3-1) in a feeder-free manner in the presence of an undifferentiated state maintenance medium. In the present specification, the term "feeder-free" refers to a method of seeding and culturing the pluripotent stem cells directly on the cell culture substrate without using cells (feeder cells) that have been inactivated by gamma irradiation or the like. By adherently culturing the pluripotent stem cells seeded in the step (3-1) in a feeder-free manner in the presence of an undifferentiated state maintenance medium, the pluripotent stem cells can proliferate in the region (A), and the embryoids layered in the out-of-plane direction of the substrate can be formed. When the undifferentiated state maintenance medium is not used, embryoids lose differentiation pluripotency, and the differentiation induction efficiency into endodermal cells deteriorates. In addition, by culturing the pluripotent stem cells in a feeder-free manner, it is possible to facilitate maintenance of nutrients in the medium during culture, enhance cell viability, and facilitate undifferentiated state maintenance. In addition, by the adherent culture, it is easier to exchange the medium than the suspension culture, and thus culture workability is excellent, and it is possible to exchange the entire amount of the medium. Therefore, the concentration of waste products discharged from the cells can be kept low. In addition, since the influence of the concentration gradient in the culture system is low, the differentiation induction efficiency is excellent.

The undifferentiated state maintenance medium is the same as in <Method for inducing differentiation into ectodermal cells>.

In the step (3-2), the ROCK inhibitor similar to the step (3-1) may be added in order to enhance the survival rate of pluripotent stem cells. The types and concentrations of suitable ROCK inhibitors are the same as described above.

In the step (3-2), from the viewpoint of suitability of enhancing the differentiation induction efficiency in the step (3-3) described later, it is preferable to perform culturing until the number of cells per unit area of the region (A) reaches 1.0 × 10⁴ cells/cm² or more, more preferably 2.0 × 10⁴ cells/cm² or more, still more preferably 5.0 × 10⁴ cells/cm² or more, and most preferably 7.5 × 10⁴ cells/cm² or more. In addition, from the viewpoint of suitability of maintaining nutrients in the medium and enhancing the survival rate of cells in the step (3-3), it is preferable to proceed to the step (3-3) when the number of cells per unit area of the region (A) is 5.0 × 10⁶ cells/cm² or less, more preferably 1.0 × 10⁶ cells/cm² or less, still more preferably 5.0 × 10⁵ cells/cm² or less, and most preferably 1.0 × 10⁵ cells/cm² or less.

In order to obtain the number of cells per unit area of the region (A), it is preferable to perform culturing in the step (3-2) for 1 to 48 hours, more preferably 6 to 36 hours, still more preferably 12 to 36 hours, and most preferably 18 to 30 hours.

The shape of the embryoids formed in the step (3-2) can be set as appropriate, and examples thereof include a sphere, an ellipsoid, a polyhedron, and a closed shape consisting of irregular planes and/or curved surfaces. From the viewpoint of suitability of enhancing the differentiation induction efficiency in the step (3-3) described later, the shape of the embryoids formed in the step (3-2) is preferably a hemispherical shape. The shape of the embryoids can be controlled by the shape of the region (A) (island shape) of the cell culture substrate.

From the viewpoint of suitability of producing embryoids having a shape close to a sphere (for example, spherical, hemispherical), the island shape is preferably circular, elliptical, or polygonal, more preferably circular, elliptical, or rectangular, particularly preferably circular, elliptical, or square, and most preferably circular or elliptical.

In addition, from the viewpoint of suitability of producing embryoids having a shape close to a sphere (for example, spherical, hemispherical), the aspect ratio of the island shape is preferably 5 or less, more preferably 2 or less, particularly preferably 1.5 or less, and most preferably 1.1 or less. Here, the "aspect ratio" in the present specification indicates major diameter/minor diameter, which is the ratio of the maximum diameter (major diameter) to the minimum diameter (minor diameter) of the shape.

The step (3-3) is a step in which the embryoids formed in the step (3-2) are cultured in the presence of a medium comprising a differentiation-inducing factor in a state of adhering onto the culture surface of the cell culture substrate, and induced to differentiate to form endodermal cell aggregates.

Differentiation-inducing factors include, for example, TGF-β inhibitors, ATP competitive inhibitors, and GSK3 inhibitors. Differentiation-inducing factors may be used singly or in combination of two or more.

From the viewpoint of suitability of enhancing the differentiation induction efficiency into endodermal cells, the differentiation-inducing factor in the step (3-3) preferably comprises an endoderm-inducing factor. As the endoderm-inducing factor, from the viewpoint of enhancing the differentiation induction efficiency, at least one differentiation-inducing factor selected from the group consisting of Wnt protein, bone morphogenetic protein (BMP), insulin-like growth factor, and activin are preferable, and it is particularly preferable to contain any one of Wnt3a, BMP4, IGFI, and activin A.

In addition, the concentration of the differentiation-inducing factor added to the medium is not particularly limited, and from the viewpoint of enhancing the differentiation induction efficiency, the concentration is preferably 1000 to 500 ng/mL, more preferably 500 to 100 ng/mL, and most preferably 100 ng/mL or less.

The medium comprising the differentiation-inducing factor is preferably exchanged every 24 hours during the culture such that the differentiation induction proceeds sufficiently. By exchanging the medium within 24 hours, it is possible to prevent a shortage of differentiation-inducing factors in the medium and to uniformly differentiate all the cells.

Endoderm markers possessed by the endodermal cells formed in step (3-3) are preferably FOXA2, CXCR4, NKX2.1, AFP, SERPINA1, SST, ISL1, IPF1, IAPP, PAX4, and TAT, and most preferably SOX17.

In the step (3-3), embryoids are cultured in a state of adhering onto the culture surface of the cell culture substrate. By inducing the differentiation of the embryoids formed in the step (3-2) in a state of adhering onto the culture surface of the cell culture substrate, the concentration gradient of the above-described differentiation-inducing factor has little effect, and thus the uniform differentiation is expected to proceed. The medium used in the step (3-3) is not particularly limited, but examples thereof include a culture medium obtained by adding the endoderm-inducing factor and a culture supplement to a basal medium such as DMEM, Ham's F12, and D-MEM/Ham's F12.

The types of cells induced to differentiate from the embryoids in the step (3-3) are not particularly limited except that the cells are endodermal cells, but for example, thyroid cells and urinary tract cells are preferable, and gastric epithelial cells, liver cells, pancreatic cells, and intestinal epithelial cells are more preferable from the viewpoint of using for regenerative medicine and drug discovery.

The intestinal epithelial cells comprising at least one type of cell selected from the group consisting of enterocytes, goblet cells, enteroendocrine cells, Paneth cells, and intestinal epithelial stem cells is preferable, and it is more preferable to have all of enterocytes, goblet cells, enteroendocrine cells, Paneth cells, and intestinal epithelial stem cells. Intestinal epithelial cells have unique markers. Examples thereof include CDX2 as an intestinal cell marker, VIL1 as an absorptive epithelial cell marker, MUC2 as an embryonic cell marker, CGA as an enteroendocrine cell marker, DEFA6 as a Paneth cell marker, and LGR5 as an intestinal epithelial stem cell marker.

The shape of the endodermal cell aggregates obtained in the step (3-3) is usually the same as the shape of the embryoids formed in the step (3-2). Specific examples of the shape of the aggregate include a shape close to a sphere (for example, spherical, hemispherical).

### <Kit for inducing differentiation into trigeminal cells>

A kit for inducing differentiation into trigeminal cells, including a cell culture substrate comprising a region (A) and a region (B) may further include a single or a plurality of substances (substrates) selected from the group consisting of Matrigel, laminin, fibronectin, vitronectin, collagen, and fragments thereof, may further include a medium comprising a differentiation-inducing factor (differentiation-inducing medium), or may further include a substrate and a differentiation-inducing medium.
(A) An island-shaped region with an area of 0.001 to 5 mm² having cell adhesiveness and cell proliferation.
(B) A region adjacent to the region (A) and having no cell adhesiveness or having no cell proliferation.

It is assumed that, before using the kit for differentiation induction, a composition comprising the above-described substrate was added to the cell culture substrate having the following region (A) and the following region (B), based on the area of the culture surface of the cell culture substrate such that the total amount of substrate is 1 to 100 µg/cm².

Matrigel, laminin, fibronectin, vitronectin, collagen, and fragments thereof are the same as in <Method for inducing differentiation into ectodermal cells>.

The term "trigeminal cell" is a general term for ectodermal cells, mesodermal cells, and endodermal cells. Any of ectodermal cells, mesodermal cells, and endodermal cells can be induced to differentiate by properly using the differentiation-inducing factor used together with the kit for inducing differentiation or the differentiation-inducing factor included in the kit for inducing differentiation.

When the purpose is to induce differentiation into ectodermal cells, the ectoderm-inducing factor exemplified in <Method for inducing differentiation into ectodermal cells> can be used at concentrations added to the medium as exemplified in <Method for inducing differentiation into ectodermal cells>. When the purpose is to induce differentiation into mesodermal cells, the mesoderm-inducing factor exemplified in <Method for inducing differentiation into mesodermal cells> can be used at concentrations added to the medium as exemplified in <Method for inducing differentiation into mesodermal cells>. When the purpose is to induce differentiation into endodermal cells, the endoderm-inducing factor exemplified in <Method for inducing differentiation into endodermal cells> can be used at concentrations added to the medium as exemplified in <Method for inducing differentiation into endodermal cells>.

### [Example]

Hereinafter, the method for inducing differentiation into ectodermal cells according to the present invention will be described in detail with reference to the mode for carrying out the present invention, but the present invention is an example for description, and is not intended to limit the present invention to the following contents. Moreover, appropriate modification is possible within the scope of the gist of the present invention. In addition, commercially available reagents were used unless otherwise specified.

### <Diameter and aspect ratio of embryoid bodies (embryoids)>

Using an inverted phase-contrast microscope (manufactured by Olympus Corporation, model number: IX73) and a digital camera (manufactured by Olympus Corporation, model number: DP73), the embryoid bodies (embryoids) were observed over time, and the major and minor axis lengths were measured. The aspect ratio (major axis/minor axis) was calculated from the obtained major axis and minor axis lengths and used to evaluate the embryoid body (embryoid) morphology. In addition, in the example of the present invention, a circular pattern is formed, and the aspect ratio is theoretically 1.0.

### [Example 1]

### <Composition of differentiation-inducing medium and like>

Differentiation-inducing medium 1: 80% DMEM/F-12 (Fujifilm Wako Pure Chemical Corporation), 20% KnockOut Serum Replacement XenoFree (manufactured by Thermo Fisher Scientific Inc.), 0.1 mM non-essential amino acids (manufactured by Sigma-Aldrich), 0. 1 mM 2-mercaptoethanol (manufactured by Sigma-Aldrich), 4 ng/mL basic fibroblast growth factor (bFGF), 3 µM SB431542 (manufactured by Fujifilm Wako Pure Chemical Corporation), 3 µM dorsomorphin (manufactured by Sigma-Aldrich), 3 µM CHIR99021 (manufactured by REPRO CELL Inc.). Differentiation-inducing medium 2: 98% KBM serum-free medium for neural stem cell culture (Fujifilm Wako Pure Chemical Corporation), 2% B27 supplement (manufactured by Invitrogen), 20 ng/mL bFGF, 10 ng/mL recombinant human-derived LIF (hLIF, manufactured by Merck & Co., Inc.), 2 µM SB431542, 3 µM CHIR99021, 2 µM retinoic acid (manufactured by Sigma-Aldrich), 1 µM purmorphamine (manufactured by Merck & Co., Inc.). Differentiation-inducing medium 3: 98% KBM neural stem cell culture serum-free medium, 2% B27 supplement, 2 ng/mL bFGF, 10 ng/mL hLIF, 2 µM SB431542, 3 µM CHIR99021, 2 µM retinoic acid, 1 µM purmorphamine, 5 µM DAPT (added on the "differentiation day 16," manufactured by Sigma-Aldrich). Cell dissociation Solution: 1:1 mixture of TrypLE select (manufactured by Gibco) and 0.5 mM EDTA solution (manufactured by Invitrogen). Membrane permeation solution: 0.3% TritonX-100 (Fujifilm Wako Pure Chemical Corporation), 1% bovine serum albumin-comprising PBS(-) (Fujifilm Wako Pure Chemical Corporation) solution blocking solution, antibody diluent. Flow cytometry analysis buffer: 1% BSA-comprising PBS(-) solution.

### [Example 1]

### <Preparation of pluripotent stem cell aggregates>

A cell culture substrate having a cell adhesive and cell proliferation region was prepared by covering a dish (manufactured by Sumitomo Bakelite Co., Ltd., product name: PrimeSurface (registered trademark)) with a diameter of 35 mm coated with a hydrophilic polymer on the surface with a metal mask (manufactured by Mitani Micronics Co., Ltd.) comprising a plurality of circular holes with a diameter of 0.2 mm, and by performing plasma treatment (under 20 Pa gas pressure, conductive current 20 mA, irradiation time 30 seconds) from above the metal mask using a plasma irradiation device (manufactured by Vacuum Device Co., Ltd., product name: Plasma Ion Bombarder PIB-20).

2.0 mL/dish of StemFit AK02N (manufactured by Ajinomoto Co., Inc.), which is an undifferentiated state maintenance medium, was added to the patterned cell culture substrate, and 3900 cells/cm² of human iPS cell 201B7 strain and iMatrix-511 solution (manufactured by Nippi, Inc.) were added at a concentration of 2.5 µL/mL. The cells were cultured at 37°C and a CO₂ concentration of 5%. In addition, Y-27632 (manufactured by Fujifilm Wako Pure Chemical Corporation) (concentration 10 µM) was added to the medium until 24 hours after seeding the cells. A plurality of hemispherical cell aggregates adhering onto the cell culture substrate were confirmed by phase-contrast microscopic observation.

### <Preparation of embryoid bodies (embryoids)>

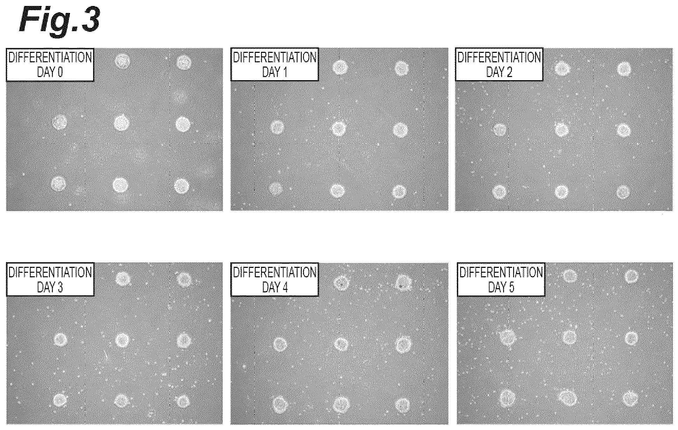

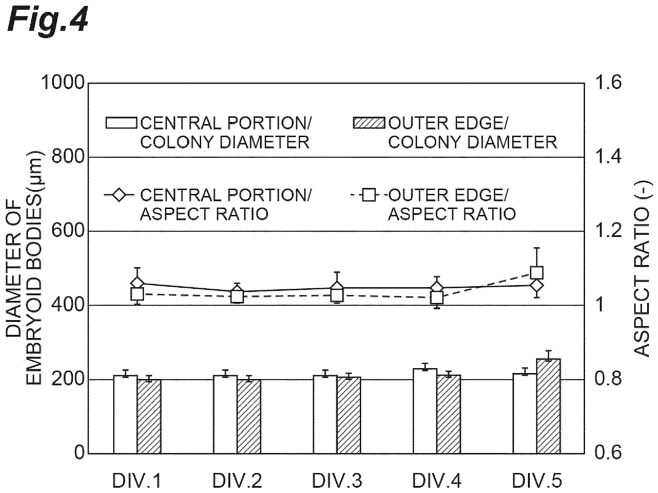

2.0 mL/dish of differentiation-inducing medium 1 was added 24 hours after seeding the cells to initiate differentiation induction. FIG. 3 is phase-contrast micrographs of embryoid bodies on the cell culture substrate in Example 1. The time point at which the differentiation-inducing medium 1 was added was defined as "differentiation day 0." After that, the differentiation-inducing medium 1 was exchanged every 24 hours of culture until the "differentiation day 5." In addition, it was confirmed that, when the medium was exchanged, the major axis and minor axis of the embryoid body (embryoid) were measured using a phase-contrast microscope according to the procedure described above, and the embryoid body (embryoid) with a diameter of 0.2 mm, which is the hole size of the metal mask, was uniformly formed. FIG. 4 is a graph showing temporal changes in diameter and aspect ratio of embryoid bodies (embryoids) in Example 1. The vertical axis (left) in FIG. 4 indicates the diameter of the embryoid body (µm), the vertical axis (right) indicates the aspect ratio (major diameter/minor diameter, which is the ratio of the maximum diameter (major diameter) to the minimum diameter (minor diameter) of the shape), and the horizontal axis indicates the number of days from differentiation day 0 to differentiation day 5. In addition, in FIG. 4, the central portion means a part including the center of the cell adhesion surface in the dish, and the outer edge means a part in the dish including the boundary between the cell adhesion surface and the side surface of the dish. As can be seen from FIG. 4, the aspect ratio was maintained at 1.0 until the differentiation day 5, the shape of the embryoid body (embryoid) was uniform, and thus it was suggested that differentiation progress of cells that form embryoid bodies (embryoids) was synchronized.

### <Preparation of ectodermal cell aggregates>

After differentiation was induced up to "differentiation day 5," the differentiation-inducing medium 1 was removed from the dish, and the differentiation-inducing medium 2 was added at 2.0 mL/dish. After that, the differentiation-inducing medium 2 was exchanged every 48 hours of culture until the "differentiation day 12." On the "differentiation day 10," the release of embryoid bodies (embryoids) from the substrate surface was observed. On the "differentiation day 12," the differentiation-inducing medium 2 in the dish was removed, and 2.0 mL/dish of the differentiation-inducing medium 3 was added. After that, the differentiation-inducing medium 3 was exchanged every 48 hours of culture until the "differentiation day 19."

### <Cell number measurement of embryoid bodies (embryoids)>

On the "differentiation day 5," the differentiation-inducing medium 1 in the dish was removed, and 2.0 mL/dish of the PBS(-) was added for washing. After washing, 1.0 mL/dish of cell dissociation solution was added to the dish, and the dish was allowed to stand for 3 minutes in an environment of 37°C and a CO₂ concentration of 5%. After allowing to stand for a predetermined time, the cell dissociation solution was removed, 1.0 mL/dish of differentiation-inducing medium 1 comprising Y-27632 (concentration 10 µM) was added, and the embryoid bodies (embryoids) were dissociated and dispersed from above the substrate using a cell scraper (manufactured by Iwaki Co., Ltd.). The obtained cell suspension was collected, and the number of cells was measured using a Luna automated cell counter (manufactured by Logos Biosystems, Inc.).

### <Cell number measurement of ectodermal cell aggregates>

On the "differentiation day 19," the differentiation-inducing medium 3 in the dish was removed, and 2.0 mL/dish of the PBS(-) was added for washing. After washing, 1.0 mL/dish of cell dissociation solution was added to the dish, and the dish was allowed to stand for 3 minutes in an environment of 37°C and a CO₂ concentration of 5%. After allowing to stand for a predetermined time, the cell dissociation solution was removed, 1.0 mL/dish of differentiation-inducing medium 1 comprising Y-27632 (concentration 10 µM) was added, and the embryoid bodies (embryoids) were dissociated and dispersed from above the substrate using a cell scraper (manufactured by Iwaki Co., Ltd.). The obtained cell suspension was collected, and the number of cells was measured using a Luna automated cell counter (manufactured by Logos Biosystems, Inc.).

### <Flow cytometry analysis>

In order to evaluate the differentiation induction into trigeminal bodies (embryoids), neural stem cells, and neural cells, ectoderm marker (PAX6), endoderm marker (FOXA2), mesoderm marker (TBX1), neural stem cell marker (SOX1), and nerve cell markers (NCAM1 and TUBB3) were evaluated. Cells on the "differentiation day 5" and the "differentiation day 19", which were collected by the cell number measurement, were fractionated at 1.0 × 10⁵ cells/tube respectively into 1.5 mL sample tubes. 0.5 mL/tube of 4% paraformaldehyde (manufactured by Fujifilm Wako Pure Chemical Corporation) was added, dispersed, and then allowed to stand at room temperature for 20 minutes for immobilization treatment. After the immobilization treatment, centrifugation (room temperature, 800 × g, 5 minutes) was performed, the supernatant was removed, and 0.5 mL/tube of PBS(-) was added for washing. The washing operation was repeated 3 times. After washing, 0.5 mL/tube of the membrane permeation solution was added, dispersed, and then allowed to stand at room temperature for 15 minutes to perform membrane permeation treatment. After membrane permeation treatment, centrifugation was performed, the supernatant was removed, and 0.5 mL/tube of PBS(-) was added for washing.

After washing, 0.5 mL/tube of PBS(-) comprising 1% BSA was added and dispersed, and then allowed to stand at room temperature for 1 hour for blocking treatment. After blocking treatment, centrifugation was performed, the supernatant was removed, and 0.5 mL/tube of PBS(-) was added for washing. After washing, 0.1 mL/tube of diluted antibody solution was added, dispersed, and then reacted for 1 hour at room temperature in the dark. After the reaction, centrifugation was performed, the staining solution was removed, and 0.5 mL/tube of PBS(-) was added for washing. When a non-fluorescently labeled antibody was used in the antibody reaction, 0.1 mL/tube of diluted fluorescently labeled secondary antibody was subsequently added, dispersed, and then reacted for 1 hour at room temperature in the dark. After the reaction, centrifugation was performed, the staining solution was removed, and 0.5 mL/tube of PBS(-) was added for washing. After washing, 0.5 mL/tube of antibody diluent was added, and the dispersed sample was analyzed by a flow cytometer (manufactured by Nippon Becton Dickinson Co. Ltd., product name: BD Accuri C6 Plus).

Table 1 shows the results of flow cytometry analysis of cells on the "differentiation induction day 5," and Table 2 shows the results of flow cytometry analysis of cells on the "differentiation induction day 19." It was confirmed that, on the "differentiation induction day 5," 20.0% of the cells had the ectoderm marker PAX6, 26.3% of the cells had the mesoderm marker TBX1, 28.3% of the cells had the endoderm marker FOXA2, 20.8% of the cells had the endoderm marker SOX1, and an embryoid body (embryoid)-like structure was prepared. In addition, it was confirmed that, on the "differentiation induction day 19," 11.4% of the cells had the nerve cell marker NCAM1, 46.9% of the cells had the nerve cell marker TUBB3, and the differentiation induction efficiency into nerve cells was excellent.

[Table 1]

**Table 1**

| | PAX6(+) | TBX1(+) | FOXA2(+) | SOX1(+) |
|---|---|---|---|---|
| Comparative Example 3 | 3.8% | 1.8% | 0.9% | 0.4% |
| Example 1 | 20.0% | 26.3% | 28.3% | 20.8% |
| Comparative Example 2 | 17.4% | 31.3% | 39.4% | 15.4% |

[Table 2]

**Table 2**

| | PAX6(+) | SOX1(+) | NCAM1 (+) | TUBB3(+) |
|---|---|---|---|---|
| Comparative Example 3 | 3.8% | 0.4% | 2.4% | 1.7% |
| Example 1 | 1.0% | 1.3% | 11.4% | 46.9% |
| Comparative Example 2 | 0.4% | 1.1% | 10.6% | 26.1% |

### [Comparative Example 1: Undifferentiated state maintenance culture on cell culture substrate]

Culture was performed by using the undifferentiated state maintenance medium StemFit AK02N (manufactured by Ajinomoto Co., Inc.) as the medium in Example 1 instead of the differentiation-inducing mediums 1 to 3 with all other conditions the same as in Example 1, and culture was performed for 6 days (until "differentiation induction day 5" in Example 1). On the culture day 2 ("differentiation induction day 1" in Examples), the pseudopodia extension at the outer edge of the embryoid body (embryoid), which was confirmed in Example 1, was not confirmed, and it was confirmed that the undifferentiated state was maintained.

### [Comparative Example 2: Differentiation induction on polystyrene substrate for cell culture]

### <Preparation of pluripotent stem cell aggregates and preparation of embryoid bodies (embryoids)>

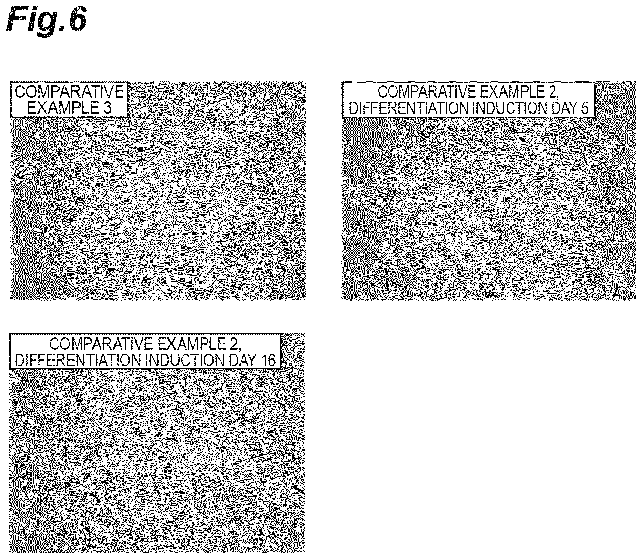

Embryoid bodies (embryoids) were prepared according to the method described in Example 1 using a commercially available culture substrate (tissue culture-treated polystyrene, manufactured by Eppendorf). The morphology of the formed structure showed an overall acute-angled shape and nonuniform size (FIG. 6).

### <Preparation of ectodermal cell aggregates>

According to the method described in Example 1, the embryoid bodies (embryoids) were induced to differentiate into ectodermal cell aggregates. It was confirmed that 100% confluency was reached on the "differentiation day 7 to 10," and a phenomenon in which the embryoid bodies (embryoids) are dissociated into a sheet when the medium was exchanged (FIG. 6) was confirmed on the "differentiation day 16."

### <Flow cytometry analysis>

Flow cytometry analysis was performed according to the method described in Example 1. Table 1 shows the results of flow cytometry analysis of cells on the "differentiation induction day 5," and Table 2 shows the results of flow cytometry analysis of cells on the "differentiation induction day 19." It was confirmed that, on the "differentiation induction day 19," 10.6% of the cells had the nerve cell marker NCAM1, 26.1% of the cells had TUBB3, and the differentiation induction efficiency into nerve cells deteriorated.

### [Comparative Example 3: Undifferentiated state maintenance culture on polystyrene substrate for cell culture]

The human iPS cells used in seeding in Example 1 were analyzed as it is by flow cytometry in the same manner as in Example 1 without performing any differentiation-inducing operation in Example 1.

Hereinafter, the method for inducing differentiation into mesodermal cells according to the present invention will be described in detail with reference to the mode for carrying out the present invention, but the present invention is an example for description, and is not intended to limit the present invention to the following contents. Moreover, appropriate modification is possible within the scope of the gist of the present invention. In addition, commercially available reagents were used unless otherwise specified.

### <Imaging of embryoid bodies (embryoids)>

Using an inverted phase-contrast microscope (manufactured by Olympus Corporation, model number: IX73) and a digital camera (manufactured by Olympus Corporation, model number: DP73), the embryoid bodies (embryoids) were observed.

### <Composition of differentiation-inducing medium and like>

Differentiation-inducing medium 1: STEMdiff Trilineage Mesoderm Medium (manufactured by Stemcell Technologies). Cell dissociation solution: 1: 1 mixture of TrypLE select (manufactured by Thermo Fisher Scientific Inc.) and 0.5 mM EDTA solution (manufactured by Invitrogen)

### [Example 2]

### <Preparation of pluripotent stem cell aggregates>

A cell culture substrate having a cell adhesive and cell proliferation region (A) was prepared by covering a dish (manufactured by Sumitomo Bakelite Co., Ltd., product name: PrimeSurface (registered trademark)) with a diameter of 35 mm coated with a hydrophilic polymer on the surface with a metal mask (manufactured by Mitani Micronics Co., Ltd.) having a plurality of circular holes with a diameter of 0.2 mm, and by performing plasma treatment (under 20 Pa gas pressure, conductive current 20 mA, irradiation time 30 seconds) from above the metal mask using a plasma irradiation device (manufactured by Vacuum Device Co., Ltd., product name: Plasma Ion Bombarder PIB-20).

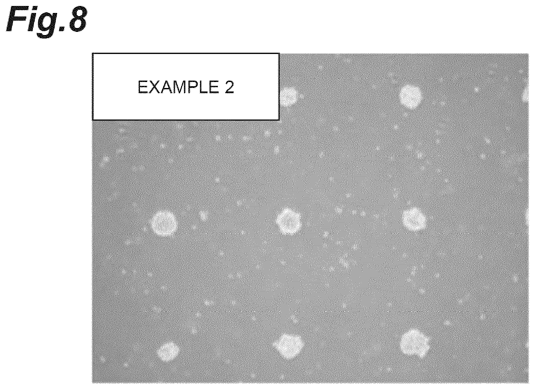

2.0 mL/dish of StemFit AK02N (manufactured by Ajinomoto Co., Inc.), which is an undifferentiated state maintenance medium, was added to the cell culture substrate, and 3900 cells/cm² of human iPS cell 201B7 strain and iMatrix-511 solution (manufactured by Nippi, Inc.) were added at a concentration of 2.5 µL/mL. The cells were cultured at 37°C and a CO₂ concentration of 5%. In addition, Y-27632 (manufactured by Fujifilm Wako Pure Chemical Corporation) (concentration 10 µM) was added to the medium until 24 hours after seeding the cells. A plurality of circular embryoid bodies (embryoids) adhering onto the cell culture substrate were confirmed by phase-contrast microscopic observation (FIG. 8).

### <Preparation of mesodermal cell aggregates>

2.0 mL/dish of differentiation-inducing medium 1 was added 24 hours after seeding the cells to initiate differentiation induction. The time point at which the differentiation-inducing medium 1 was added was defined as "differentiation day 0." After that, the differentiation-inducing medium 1 was exchanged every 24 hours of culture until the "differentiation day 6."

### <Cell number measurement of mesodermal cell aggregates>

On the "differentiation day 6," the differentiation-inducing medium 1 in the dish was removed, and 2.0 mL/dish of the PBS(-) was added for washing. After washing, 1.0 mL/dish of cell dissociation solution was added to the dish, and the dish was allowed to stand for 5 minutes in an environment of 37°C and a CO₂ concentration of 5%. After allowing to stand for a predetermined time, the cell dissociation solution was removed, 1.0 mL/dish of differentiation-inducing medium 1 was added, and the embryoid bodies (embryoids) were dissociated and dispersed from above the substrate using a cell scraper (manufactured by Iwaki Co., Ltd.). The obtained cell suspension was collected, and the number of cells was measured using a Luna automated cell counter (manufactured by Logos Biosystems, Inc.).

### <Gene expression analysis>

The mesoderm marker (BRACHYURY) and the undifferentiated marker (NANOG) were evaluated in order to evaluate differentiation induction into mesodermal cells. Cells on the "differentiation day 6", which were collected by the cell number measurement, were fractionated at 1.0 × 10⁶ cells/tube respectively into 1.5 mL sample tubes. Centrifugation (room temperature, 800 × g, 5 minutes) was performed and the supernatant was removed. RNA was extracted from the cells using RNeasy Plus Mini Kit (manufactured by QIAGEN). The concentration of the extracted RNA was measured with a Qubit4 Fluorometer, and adjusted to 1 µg/6 µL with RNase-free Water (manufactured by Takara Bio Inc.). For the reverse transcription reaction, ReverTra Ace qRT Master Mix with gDNA Remover (manufactured by Toyobo Co., Ltd.) was used. For 1 µL of 5-fold diluted cDNA (equivalent to 1/100 of the reverse transcription product), by using oligo dT primers (manufactured by Integrated DNA Technologies) having gene sequences (BRACHYURY: SEQ ID NO: 4, NANOG: SEQ ID NO: 2, GAPDH: SEQ ID NO: 3) shown in Table 3 below and THUNDERBIRD Probe qPCR Mix (manufactured by Toyobo Co., Ltd.), real-time PCR analysis was performed using a QuantStudio3 real-time PCR system (manufactured by Thermo Fisher Scientific Inc.).

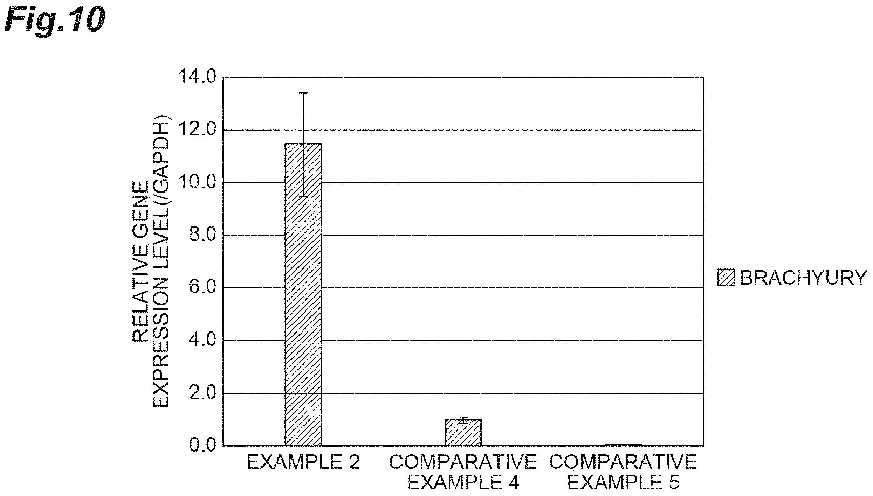

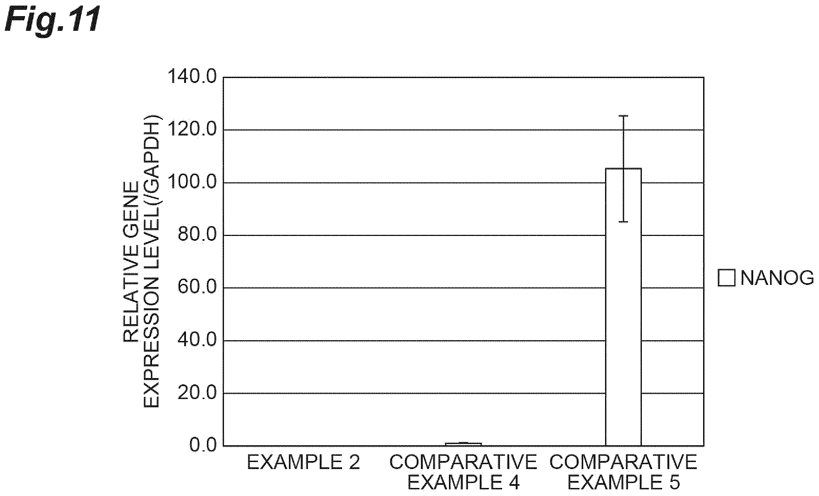

The results of gene expression analysis of cells on the "differentiation induction day 6" are shown in FIGS. 10 and 11. The vertical axis in FIG. 10 indicates the relative gene expression level of BRACHYURY with respect to GAPDH, and the vertical axis in FIG. 11 indicates the relative gene expression level of NANOG with respect to GAPDH. In both graphs, the horizontal axis is Example 2, Comparative Example 4, or Comparative Example 5. From FIGS. 10 and 11, on the "differentiation induction day 6" the preparation of an embryoid body (embryoid)-like structure was confirmed, and it was confirmed that the cells lost the undifferentiated marker NANOG and had the mesoderm marker BRACHYURY. In addition, patterning the cells increased the expression level of BRACHYURY, and thus it was confirmed that adherent culture in patterning significantly promoted differentiation.

**[Table 3]**

| Primer | Sequence (5'→3') |
|---|---|
| BRACHYURY | CGTCTCCTTCAGCAAAGTCAA CTATGTGGATTCGAGGCTCATAC |
| NANOG | AACTCTCCAACATCCTGAACC CCTTCTGCGTCACACCATT |
| GAPDH | ACATCGCTCAGACACCATG TGTAGTTGAGGTCAATGAAGGG |

[Comparative Example 4: Differentiation induction on polystyrene substrate for cell culture]

### <Monolayer culture of pluripotent stem cell aggregates>

2.0 mL/dish of StemFit AK02N (manufactured by Ajinomoto Co., Inc.), which is an undifferentiated state maintenance medium, was added to a commercially available culture substrate (tissue culture-treated polystyrene, manufactured by Eppendorf), and 5000 cells/cm² of human iPS cell 201B7 strain and iMatrix-511 solution (manufactured by Nippi, Inc.) were added at a concentration of 2.5 µL/mL. The cells were cultured at 37°C and a CO₂ concentration of 5%. In addition, Y-27632 (manufactured by Fujifilm Wako Pure Chemical Corporation) (concentration 10 µM) was added to the medium until 24 hours after seeding the cells.

### <Differentiation induction into mesodermal cells>

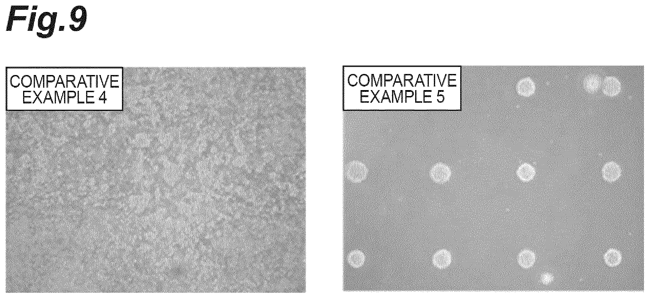

The monolayer-cultured cells were induced to differentiate into mesodermal cells using differentiation-inducing medium 1 for 6 days. FIG. 9 (drawing on the left) shows the state on the differentiation induction day 6. As can be seen from FIG. 9, the cells adhered to the entire substrate and did not form spherical embryoids.

### <Gene expression analysis>

Gene expression analysis was performed according to the method described in Example 2.

### [Comparative Example 5: Undifferentiated state maintenance culture on polystyrene substrate for cell culture]

### <Preparation of pluripotent stem cell aggregates and preparation of embryoid bodies (embryoids)>

Embryoid bodies (embryoids) were prepared according to the method described in Example 2 using the same substrate as in Example 2.

### <Undifferentiated state maintenance culture>

Undifferentiated state maintenance culture was performed with respect to embryoid bodies (embryoids) using StemFitAK02N for 6 days.

FIG. 9 (drawing on the right) shows the state after 6 days of undifferentiated state maintenance culture.

### <Gene expression analysis>

Gene expression analysis was performed according to the method described in Example 2.

Hereinafter, the method for inducing differentiation into endodermal cells according to the present invention will be described more specifically based on examples. However, the present invention is not limited to the following examples. In addition, commercially available reagents were used unless otherwise specified.

### <Measurement of area of region (A)>

Using a laser microscope (manufactured by Keyence Corporation, product name VK-X200), an image of the surface of the cell culture substrate was acquired. Using the obtained image, the area of the region (A) at 20 points was obtained using analysis software (VK-X Viewer), and these areas were averaged to obtain the area of the region (A).

### <Imaging of cells and cell aggregates>

Using an inverted phase-contrast microscope (manufactured by Olympus Corporation, model number: IX73) and a digital camera (manufactured by Olympus Corporation, model number: DP73), phase-contrast microscopic images and trend microscopic images of cells and aggregates of cells were taken.

### <Composition of differentiation-inducing medium and like>

Differentiation-inducing medium 1: STEMdiff Trilineage Mesoderm Medium (manufactured by STEMCELL Technologies)

Differentiation-inducing medium 2: 85% KnockOut DMEM (manufactured by Thermo Fisher Scientific Inc.), 15% KnockOut Serum Replacement XenoFree (manufactured by Thermo Fisher Scientific Inc.), 0.1 mM non-essential amino acids (manufactured by Sigma-Aldrich), 2 mM Gluta Max-I Supplement (manufactured by Thermo Fisher Scientific Inc.), 20 ng/mL basic fibroblast growth factor (bFGF, manufactured by PeproTech, Inc.), 50 µg/mL L(+)-ascorbic acid (Fujifilm Wako Pure Chemical Corporation), 10 ng/mL Heregulin-β-1 (Fujifilm Wako Pure Chemical Corporation), 200 ng/mL Long (R) R3IGF-I (manufactured by Sigma-Aldrich), 1% penicillin-streptomycin solution (x 100) (Fujifilm Wako Pure Chemical Corporation)

Cell dissociation solution: 1:1 mixture of TrypLE select (manufactured by Thermo Fisher Scientific Inc.) and 0.5 mM EDTA solution (manufactured by Invitrogen)

### [Example 3]

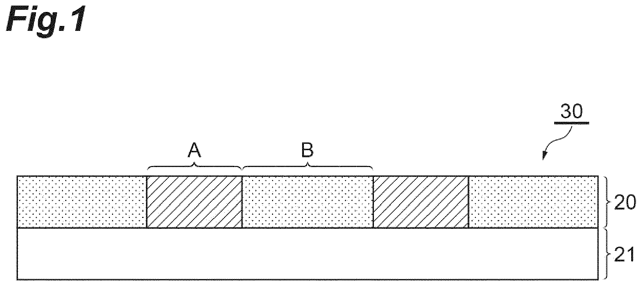

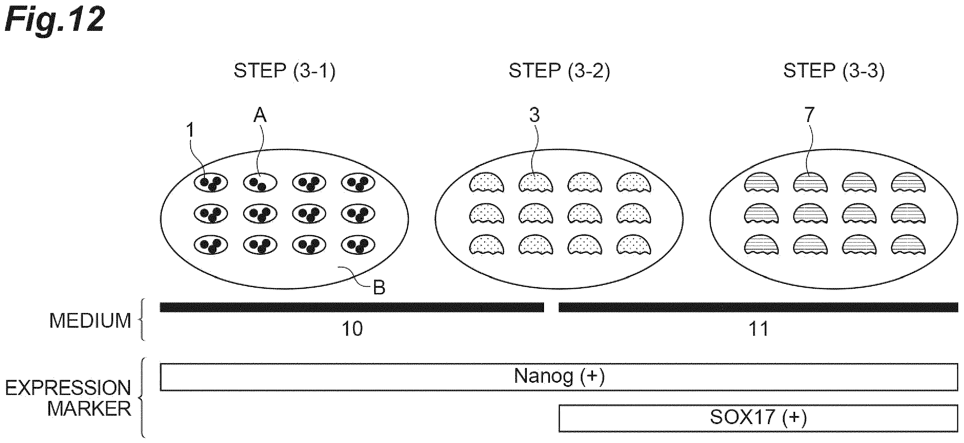

FIG. 12 is a diagram showing an outline of a culture method. FIG. 1 is a schematic diagram (sectional view) of a cell culture substrate. Hereinafter, description will be made with reference to FIGS. 1 and 12 as necessary.

### <Preparation of pluripotent stem cell aggregates>

A cell adhesive and cell proliferation region (region (A)) was formed at the plasma-treated part by covering a dish (manufactured by Sumitomo Bakelite Co., Ltd., product name: PrimeSurface (registered trademark)) with a diameter of 35 mm coated with a hydrophilic polymer on the surface with a metal mask (manufactured by Mitani Micronics Co., Ltd.) having a plurality of circular holes with a diameter of 0.2 mm, and by performing plasma treatment (under 20 Pa gas pressure, conductive current 20 mA, irradiation time 30 seconds) from above the metal mask using a plasma irradiation device (manufactured by Vacuum Device Co., Ltd., product name: Plasma Ion Bombarder PIB-20). In addition, a region (B) was formed at the part masked with a metal mask (a part not subjected to plasma treatment). The outline of the obtained cell culture substrate is as shown in the schematic diagram of FIG. 1. That is, in the cell culture substrate 30 shown in FIG. 1, the surface of a substrate 21 is coated with a hydrophilic polymer 20, and further, the hydrophilic polymer 20 is decomposed at the plasma-treated part, and the region (A) indicated by A is formed. In addition, at the part not subjected to the plasma treatment, the hydrophilic polymer 20 remains as it is, and the region (B) indicated by B is formed. This was used as a cell culture substrate.

2.0 mL/dish of StemFit AK02N (manufactured by Ajinomoto Co., Inc.), which is an undifferentiated state maintenance medium (indicated by reference numeral 10 in FIG. 12), was added to the cell culture substrate, and 3900 cells/cm² of human iPS cell 201B7 strain and iMatrix-511 solution (laminin fragment solution, manufactured by Nippi, Inc.) were added to be 1.5 µg/cm² based on the area of the culture surface of the cell culture substrate (step (3-1) shown in FIG. 12). As shown in the step (3-1) of FIG. 12, the seeded human iPS cell 201B7 strain (indicated by reference numeral 1 in FIG. 12) adheres to the region (A) indicated by A, and does not adhere to the region (B) indicated by B. Then, the cells were cultured at 37°C and a CO₂ concentration of 5%. In addition, Y-27632 (manufactured by Fujifilm Wako Pure Chemical Corporation) (concentration 10 µM) was added to the medium until 24 hours after seeding the cells. A plurality of circular embryoids (indicated by reference numeral 3 in FIG. 12) adhering onto the cell culture substrate were confirmed by phase-contrast microscopic observation (step (3-2) shown in FIG. 12).

### <Preparation of endodermal cell aggregates>

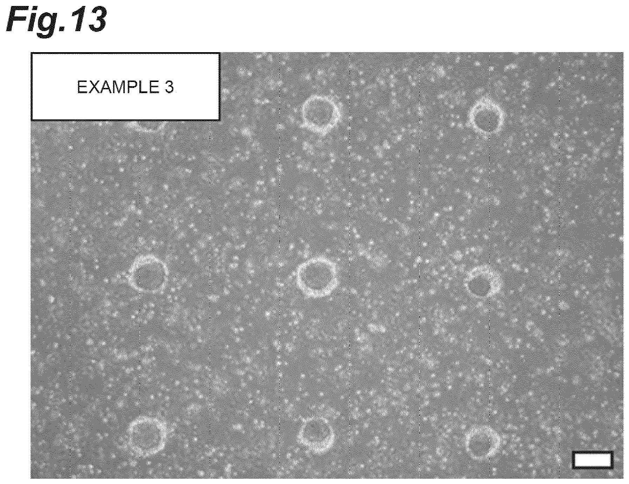

24 hours after seeding the cells, 2.0 mL/dish of the differentiation-inducing medium 1 (indicated by reference numeral 11 in FIG. 12) was added to initiate differentiation induction. The time point at which the differentiation-inducing medium 1 was added was defined as "differentiation day 0." After that, the differentiation-inducing medium 1 was exchanged every 24 hours of culture until the "differentiation day 6" (step (3-3) shown in FIG. 12). Endodermal cell aggregates (indicated by reference numeral 7 in FIG. 12) were obtained on the "differentiation day 6" (FIG. 13).

### <Cell number measurement of endodermal cell aggregates>

On the "differentiation day 6," the differentiation-inducing medium 1 in the dish was removed, and 2.0 mL/dish of the PBS(-) was added for washing. After washing, 1.0 mL/dish of cell dissociation solution was added to the dish, and the dish was allowed to stand for 5 minutes in an environment of 37°C and a CO₂ concentration of 5%. After standing, the cell dissociation solution was removed, 1.0 mL/dish of differentiation-inducing medium 1 was added, endodermal cell aggregates were dissociated from the cell culture substrate using a cell scraper (manufactured by Iwaki Co., Ltd.), and the cells were dispersed. The obtained cell suspension was collected, and the number of cells was measured using a Luna automated cell counter (manufactured by Logos Biosystems, Inc.).

### <Gene expression analysis>

In order to evaluate the differentiation induction into endodermal cells, a housekeeping gene marker (GAPDH), an undifferentiated marker (NANOG), and an endoderm marker (SOX17) were employed, and relative expression levels of mRNA of the undifferentiated marker and the endoderm marker were quantified. Cells on the "differentiation day 6", which were collected by the cell number measurement, were fractionated at 1.0 × 10⁶ cells/tube into 1.5 mL sample tubes. Centrifugation (room temperature, 800 × g, 5 minutes) was performed and the supernatant was removed. RNA was extracted from the cells using RNeasy Plus Mini Kit (manufactured by QIAGEN). The concentration of the extracted RNA was measured with a Qubit4 Fluorometer, and adjusted to 1 µg/6 µL with RNase-free Water (manufactured by Takara Bio Inc.). For the reverse transcription reaction, ReverTra Ace qRT Master Mix with gDNA Remover (manufactured by Toyobo Co., Ltd.) was used. For 1 µL of 5-fold diluted cDNA (equivalent to 1/100 of the reverse transcription product), by using oligo dT primers, primers having base sequences shown in Table 4 (manufactured by Integrated DNA Technologies), and THUNDERBIRD Probe qPCR Mix (manufactured by Toyobo Co., Ltd.), real-time PCR analysis was performed using a QuantStudio3 real-time PCR system (manufactured by Thermo Fisher Scientific Inc.).

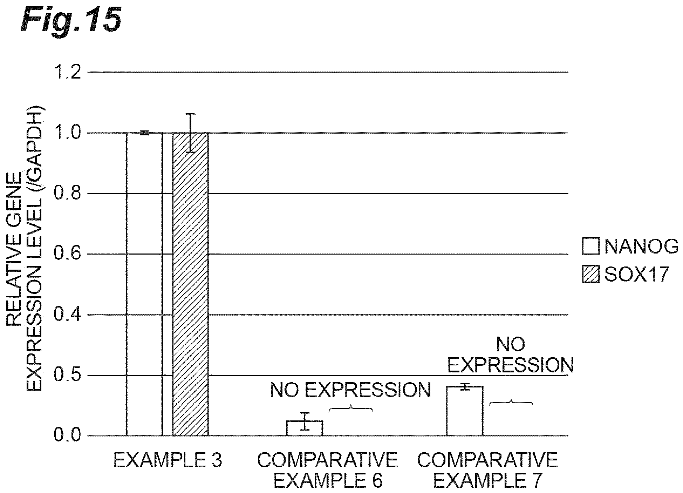

FIG. 15 is a graph showing evaluation results of gene expression levels of an undifferentiated marker (NANOG) and an endoderm marker (SOX17). The vertical axis in FIG. 15 indicates the relative gene expression level of NANOG or SOX17 with respect to GAPDH, and the horizontal axis indicates Example 3, Comparative Example 6, or Comparative Example 7. In Example 3, the formation of embryoids was confirmed, and from FIG. 15, the expression of SOX17, which is an endoderm marker, was confirmed as a result of evaluating the gene expression level in the cells on the "differentiation induction day 6."

**[Table 4]**

| Primer | SEQ ID NO | Sequence (5'->3') |
|---|---|---|
| SOXl7 | SEQ ID NO 1 | AACGCCGAGTTGAGCAAGGCCGGTACTTGTAGTTGG |
| NANOG | SEQ ID NO 2 | AACTCTCCAACATCCTGAACCCCTTCTGCGTCACACCATT |
| GAPDH | SEQ ID NO 3 | ACATCGCTCAGACACCATGTGTAGTTGAGGTCAATGAAGGG |

### [Comparative Example 6: Differentiation induction on polystyrene substrate for cell culture]

### <Monolayer culture of pluripotent stem cell aggregates>

2.0 mL/dish of StemFit AK02N (manufactured by Ajinomoto Co., Inc.), which is an undifferentiated state maintenance medium, was added to a commercially available cell culture substrate (tissue culture-treated polystyrene, manufactured by Eppendorf), and 3900 cells/cm² of human iPS cell 201B7 strain and iMatrix-511 solution (laminin fragment solution, manufactured by Nippi, Inc.) were added based on the area of the culture surface of the cell culture substrate at a concentration of 1.5 µg/cm². The cells were cultured at 37°C and a CO₂ concentration of 5%. In addition, Y-27632 (manufactured by Fujifilm Wako Pure Chemical Corporation) (concentration 10 µM) was added to the medium until 24 hours after seeding the cells.

### <Differentiation induction into endodermal cells>

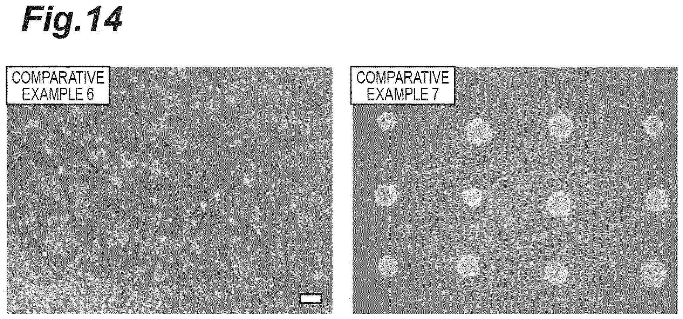

Differentiation-inducing medium 1 was added to the monolayer-cultured cells to induce differentiation into endodermal cells for 6 days. FIG. 14 (drawing on the left) shows the state on the differentiation induction day 6. As can be seen from FIG. 14 (drawing on the left), cells aggregated.

### <Gene expression analysis>

Gene expression analysis was performed according to the method described in Example 3. Results are shown in FIG. 15. In Comparative Example 6, as a result of the evaluation of the gene expression level, the expression of the endoderm marker was not confirmed, and the expression of only the undifferentiated marker was confirmed.

### [Comparative Example 7: Undifferentiated state maintenance culture on polystyrene substrate for cell culture]

### <Preparation of pluripotent stem cell aggregates>

Embryoids were prepared according to the method described in Example 3 using the same substrate as in Example 3.

### <Undifferentiated state maintenance culture>

StemFitAK02N was added to the obtained embryoids and undifferentiated state maintenance culture was performed for 6 days.

FIG. 14 (drawing on the right) shows the state on the differentiation induction day 6.

### <Gene expression analysis>

Gene expression analysis was performed according to the method described in Example 3. Results are shown in FIG. 15. In Comparative Example 7, as a result of the evaluation of the gene expression level, the expression of the endoderm marker was not confirmed, and the expression of only the undifferentiated marker was confirmed.

### [Example 4]

### <Preparation of pluripotent stem cell aggregates>

Cell adhesive and cell proliferation regions (the region (A) and the region (B)) were formed by covering a dish (manufactured by Sumitomo Bakelite Co., Ltd., product name: PrimeSurface (registered trademark)) with a diameter of 35 mm coated with a hydrophilic polymer on the surface with a metal mask (manufactured by Mitani Micronics Co., Ltd.) having a plurality of circular holes with a diameter of 0.8 mm, and by performing plasma treatment under the same conditions as in Example 3. This was used as a cell culture substrate.

2.0 mL/dish of StemFit AK02N (manufactured by Ajinomoto Co., Inc.) was added to the obtained cell culture substrate, and 24000 cells/cm² of human iPS cell 201B7 strain and iMatrix-511 solution (laminin fragment solution, manufactured by Nippi, Inc.) were added to be 1.5 µg/cm² based on the area of the culture surface of the cell culture substrate, and culturing was performed at 37°C and a CO₂ concentration of 5%. In addition, Y-27632 (manufactured by Fujifilm Wako Pure Chemical Corporation) (concentration 10 µM) was added to the medium until 24 hours after seeding the cells. A plurality of circular embryoids adhering onto the cell culture substrate were confirmed by phase-contrast microscopic observation.

### <Differentiation induction of pluripotent stem cells into intestinal epithelial cells>

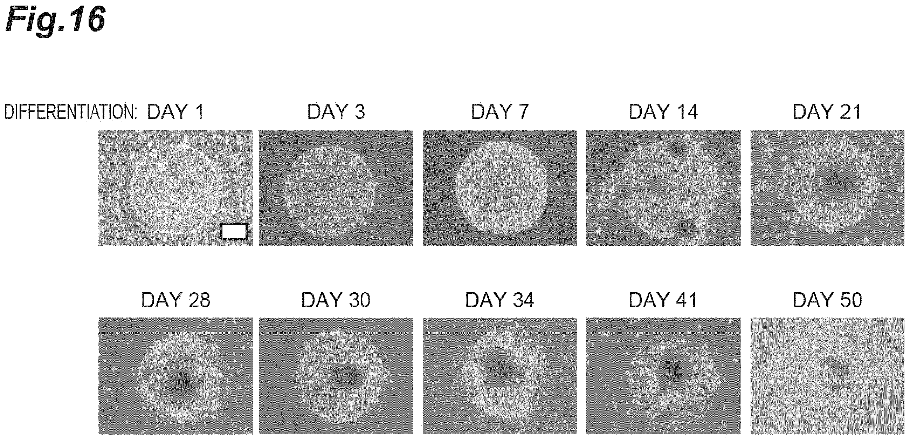

2.0 mL/dish of differentiation-inducing medium 2 was added 24 hours after seeding the cells to initiate differentiation induction. The time point at which the differentiation-inducing medium 2 was added was defined as "differentiation day 0." After that, the differentiation-inducing medium 2 was exchanged every 72 hours of culture until the "differentiation day 64." FIG. 16 is phase-contrast micrographs of cells induced to differentiate taken over time. Note that the scale bar indicates 200 µm. As shown in FIG. 16, it was confirmed that cells could be cultured in a state of adhering to the cell culture substrate from the differentiation day 0 to day 64.

### <Immunostaining analysis>

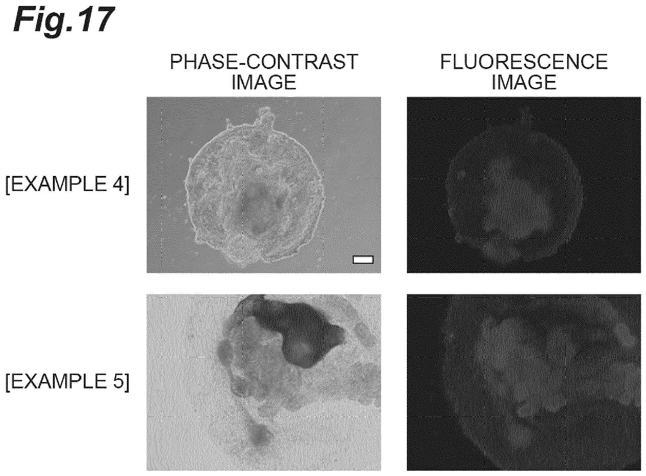

In order to evaluate the differentiation into intestinal epithelial cells, an absorptive epithelial cell marker (VILLIN) was employed, and Villin Polyclonal Antibody, Alexa Fluor 488 Conjugated (1 µg/µL, manufactured by Bioss Antibodies) was used as a fluorescence-labeled antibody. 1.0 mL/dish of 4% paraformaldehyde (manufactured by Fujifilm Wako Pure Chemical Corporation) was added to cultured cells on the differentiation day 64, and the cells were allowed to stand at room temperature for 20 minutes for immobilization treatment. After the immobilization treatment, all the solution was removed, and 1.0 mL/dish of PBS(-) was added for washing. The washing operation was repeated 3 times. After washing, 0.5 mL/dish of the membrane permeation solution was added, dispersed, and then allowed to stand at room temperature for 15 minutes to perform membrane permeation treatment. After the membrane permeation treatment, the solution was removed, and 1.0 mL/dish of PBS(-) was added for washing. After washing, 1.0 mL/dish of PBS(-) comprising 1% BSA was added and allowed to stand at room temperature for 1 hour for blocking treatment. After the blocking treatment, the solution was removed, and 1.0 mL/dish of PBS(-) was added for washing. After washing, 1.0 mL/dish of an antibody solution diluted 200 times with 1% BSA-comprising PBS(-) was added, dispersed, and then reacted for 2 hours at room temperature in the dark (antibody amount: 5.0 µg). After the reaction, the staining solution was removed, and 1.0 mL/dish of PBS(-) was added for washing. After washing, the sample to which 1.0 mL/dish of antibody diluent was added was imaged with a fluorescence microscope. The acquired fluorescence image is shown in FIG. 17. FIG. 17 is a phase-contrast micrograph of cells induced to differentiate (differentiation day 64) and a fluorescence micrograph after immunostaining. FIG. 17 also shows a phase-contrast micrograph of cells induced to differentiate in Example 5 described later and a fluorescence micrograph after immunostaining. Note that the scale bar indicates 200 µm. Cells that had been induced to differentiate for 64 days were positive for the absorptive epithelial cell marker VILLIN. That is, it was confirmed that differentiation induction of pluripotent stem cells into intestinal epithelial cells was possible in a state where the cells adhere to the cell culture substrate.

### [Example 5]

A cell culture substrate was prepared in the same manner as in Example 4, except that a metal mask having a plurality of circular holes with a diameter of 1.5 mm was used instead of a metal mask having a plurality of circular holes with a diameter of 0.8 mm. In addition, using the obtained cell culture substrate, the preparation of pluripotent stem cell aggregates and the differentiation induction of pluripotent stem cells into intestinal epithelial cells were performed in the same manner as in Example 4.

Immunostaining analysis was performed in the same manner as in Example 4 with respect to the cells on the differentiation induction day 64. As a result, as in Example 4, the cells on the differentiation induction day 64 were positive for the absorptive epithelial cell marker VILLIN, while maintaining the state of adhering to the substrate (FIG. 17). From this result, even when the diameter of the region (A) is expanded to 1.5 mm, it was confirmed that differentiation induction of pluripotent stem cells into intestinal epithelial cells was possible in a state where the cells adhere to the cell culture substrate.

### [Comparative Example 8: Examination of laminin 511-E8 fragment coating conditions]

2.0 mL/dish of StemFit AK02N (manufactured by Ajinomoto Co., Inc.), which is an undifferentiated state maintenance medium, was added to the same cell culture substrate as in Example 5, and 24000 cells/cm² of human iPS cell 201B7 strain and iMatrix-511 solution (laminin fragment solution, manufactured by Nippi, Inc.) were added to be 0.9 µg/cm² based on the area of the culture surface of the cell culture substrate. The cells were cultured at 37°C and a CO₂ concentration of 5%. In addition, Y-27632 (manufactured by Fujifilm Wako Pure Chemical Corporation) (concentration 10 µM) was added to the medium until 24 hours after seeding the cells. The day on which the cells were seeded was defined as the "culture day 0," 24 hours later was defined as the "culture day 1," and the state of the cells was imaged with a phase-contrast microscope.

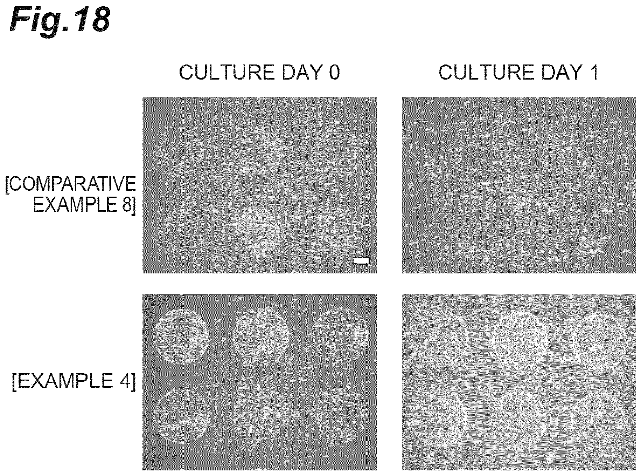

FIG. 18 is phase-contrast micrographs of cells induced to differentiate in Comparative Example 8 and Example 4. Note that the scale bar indicates 200 µm. In the cell culture substrate of Comparative Example 8, which was coated with laminin fragments (laminin 511-E8) under the condition of 0.9 µg/cm² based on the area of the culture surface of the cell culture substrate, adhesion of pluripotent stem cells was observed immediately after seeding, but cell dissociation from the adhesion region of the cells was confirmed after 24 hours. It was confirmed that, under the coating conditions of Comparative Example 8, it was difficult to culture the cells in a state where cells are maintained adherent to the cell culture substrate.

### [Comparative Example 9: Examination of coating conditions using vitronectin]

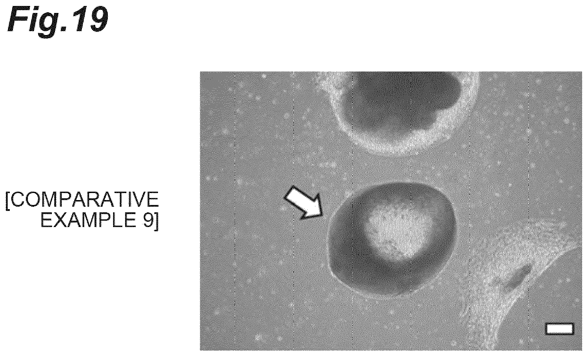

1.0 mL/dish of 1% vitronectin (VTN-N) recombinant human protein solution (manufactured by Gibco) was added to the same cell culture substrate as in Example 5, and allowed to stand at 25°C for 1 hour. After 1 hour, the 1% VTN-N solution was removed, 2.0 mL/dish of StemFit AK02N (manufactured by Ajinomoto Co., Inc.), which is an undifferentiated state maintenance medium, was added, 24000 cells/cm² of human iPS cell 201B7 strain was further added, and the differentiation induction culture was performed in the same manner as in Example 4. FIG. 19 shows the appearance of cells (phase-contrast micrograph) on the differentiation induction day 55. It was confirmed that, on the differentiation induction day 55, the cells were dissociated from the cell culture substrate. It was confirmed that, under the culture conditions of Comparative Example 9, the cells during differentiation induction could not be kept adherent to the cell culture substrate.

### Reference Signs List

A Region (A) (region having cell adhesiveness and cell proliferation)
B Region (B) (region having no cell adhesiveness or having no cell proliferation)
   1 Pluripotent stem cell
   2 Pluripotent stem cell aggregate
   3 Embryoid
   4 Ectodermal cell aggregate
   5 Nerve cell aggregate
   6 Mesodermal cell aggregate
   7 Endodermal cell aggregate
   10 Undifferentiated state maintenance medium
   11 Differentiation-inducing medium 1
   12 Differentiation-inducing medium 2
   13 Differentiation-inducing medium 3
   20 Hydrophilic polymer
   21 Substrate
   30 Cell culture substrate

## Claims

1. A method for inducing differentiation of a pluripotent stem cell into an ectodermal cell, the method comprising steps (1-1) to (1-4) below:
(1-1) a step of coating a cell culture substrate comprising two regions (A) and (B) below with a single or a plurality of substances selected from a group consisting of Matrigel, laminin, fibronectin, vitronectin, and collagen, and seeding the pluripotent stem cell,
(A) an island-shaped region with an area of 0.001 to 5 mm² having cell adhesiveness and cell proliferation, and
(B) a region adjacent to the region (A) and having no cell adhesiveness or having no cell proliferation;
(1-2) a step of forming a pluripotent stem cell aggregate adhering onto the cell culture substrate by adherently culturing the pluripotent stem cell seeded in the step (1-1) in a feeder-free manner in the presence of an undifferentiated state maintenance medium;
(1-3) a step of forming an embryoid body by culturing the pluripotent stem cell aggregate formed in the step (1-2) in the presence of a medium comprising a differentiation-inducing factor in a state of adhering onto the cell culture substrate; and
(1-4) a step of forming an ectodermal cell aggregate by culturing the embryoid body formed in the step (1-3) in the presence of a medium comprising a differentiation-inducing factor in a state of adhering onto the cell culture substrate.

2. The method for inducing differentiation according to claim 1, wherein
the cell culture substrate has a layer comprising a hydrophilic polymer on a surface thereof, and the region (A) is a region obtained by decomposing or modifying a part of the layer comprising the hydrophilic polymer by plasma treatment, ultraviolet treatment, corona discharge treatment, or a combination thereof.

3. The method for inducing differentiation according to claim 1 or 2, wherein
the differentiation-inducing factor in the step (1-3) comprises an ectoderm-inducing factor, a mesoderm-inducing factor, and an endoderm-inducing factor.

4. The method for inducing differentiation according to any one of claims 1 to 3, wherein
the ectodermal cell aggregate formed in the step (1-4) comprises PAX6 and SOX1.

5. The method for inducing differentiation according to any one of claims 1 to 4, wherein
the ectodermal cell is a neural stem cell or a nerve cell.

6. The method for inducing differentiation according to any one of claims 1 to 5, wherein
the ectodermal cell is a motor nerve cell.

7. A method for producing a cell obtained by differentiation induction of a pluripotent stem cell into an ectodermal cell, the method comprising steps (1-1) to (1-4) below:
(1-1) a step of coating a cell culture substrate comprising two regions (A) and (B) below with a single or a plurality of substances selected from a group consisting of Matrigel, laminin, fibronectin, vitronectin, and collagen, and seeding the pluripotent stem cell,
(A) an island-shaped region with an area of 0.001 to 5 mm² having cell adhesiveness and cell proliferation, and
(B) a region adjacent to the region (A) and having no cell adhesiveness or having no cell proliferation;
(1-2) a step of forming a pluripotent stem cell aggregate adhering onto the cell culture substrate by adherently culturing the pluripotent stem cell seeded in the step (1-1) in a feeder-free manner in the presence of an undifferentiated state maintenance medium;
(1-3) a step of forming an embryoid body by culturing the pluripotent stem cell aggregate formed in the step (1-2) in the presence of a medium comprising a differentiation-inducing factor in a state of adhering onto the cell culture substrate; and
(1-4) a step of forming an ectodermal cell aggregate by culturing the embryoid body formed in the step (1-3) in the presence of a medium comprising a differentiation-inducing factor in a state of adhering onto the cell culture substrate.

8. A method for inducing differentiation of a pluripotent stem cell into a mesodermal cell, the method comprising steps (2-1) to (2-3) below:
(2-1) a step of coating a cell culture substrate comprising two regions (A) and (B) below with a single or a plurality of substances selected from a group consisting of Matrigel, laminin, fibronectin, vitronectin, and collagen, and seeding the pluripotent stem cell,
(A) an island-shaped region with an area of 0.001 to 5 mm² having cell adhesiveness and cell proliferation, and
(B) a region adjacent to the region (A) and having no cell adhesiveness or having no cell proliferation;
(2-2) a step of forming an embryoid body adhering onto the cell culture substrate by adherently culturing the pluripotent stem cell seeded in the step (2-1) in a feeder-free manner in the presence of an undifferentiated state maintenance medium; and
(2-3) a step of forming a mesodermal cell aggregate by culturing the embryoid body formed in the step (2-2) in the presence of a medium comprising a differentiation-inducing factor in a state of adhering onto the cell culture substrate.

9. The method for inducing differentiation according to claim 8, wherein
the cell culture substrate has a layer comprising a hydrophilic polymer on a surface thereof, and the region (A) is a region obtained by decomposing or modifying the layer comprising the hydrophilic polymer by plasma treatment, ultraviolet treatment, and/or corona discharge treatment.

10. The method for inducing differentiation according to claim 8 or 9, wherein
a cell seeding density in the step (2-1) is 1.0 × 10² to 1.0 × 10⁶ cells/cm².

11. The method for inducing differentiation according to any one of claims 8 to 10, wherein
in the step (2-2), the embryoid body is cultured until the number of cells per unit area of the region (A) reaches 1.0 × 10⁴ cells/cm² or more.

12. The method for inducing differentiation according to any one of claims 8 to 11, wherein
a shape of the embryoid body formed in the step (2-2) is an island shape.

13. The method for inducing differentiation according to any one of claims 8 to 12, wherein
the differentiation-inducing factor in the step (2-3) comprises a mesoderm-inducing factor.

14. The method for inducing differentiation according to claim 13, wherein
the mesoderm-inducing factor is single or a plurality of differentiation-inducing factors selected from a group consisting of a GSK3β inhibitor, bone morphogenetic protein, and activin.

15. The method for inducing differentiation according to any one of claims 8 to 14, wherein
the embryoid body formed in the step (2-2) comprises a mesoderm marker.

16. A method for inducing differentiation of a pluripotent stem cell into an endodermal cell, the method comprising:
(3-1) a step of adding a composition comprising at least one selected from laminin and a fragment thereof to a cell culture substrate comprising a region (A) below and a region (B) below based on an area of a culture surface of the cell culture substrate such that a total amount of laminin and the fragment thereof is 1 to 100 µg/cm², and seeding the pluripotent stem cell,
(A) an island-shaped region with an area of 0.001 to 5 mm² having cell adhesiveness and cell proliferation, and
(B) a region adjacent to the region (A) and having no cell adhesiveness or having no cell proliferation;
(3-2) a step of forming an embryoid adhering onto the culture surface of the cell culture substrate by adherently culturing the pluripotent stem cell seeded in the step (3-1) in a feeder-free manner in the presence of an undifferentiated state maintenance medium; and
(3-3) a step of forming an endodermal cell aggregate by culturing the embryoid formed in the step (3-2) in the presence of a medium comprising a differentiation-inducing factor in a state of adhering onto the culture surface of the cell culture substrate, and by performing differentiation induction.

17. The method for inducing differentiation according to claim 16, wherein
the endodermal cell induced to differentiate in the step (3-3) expresses SOX17, which is an endoderm marker.

18. The method for inducing differentiation according to claim 16 or 17, wherein
the endodermal cell induced to differentiate in the step (3-3) is an intestinal epithelial cell.

19. The method for inducing differentiation according to claim 18, wherein
the intestinal epithelial cell induced to differentiate in the step (3-3) comprises an absorptive epithelial cell marker VIL1.

20. The method for inducing differentiation according to any one of claims 16 to 19, wherein
the cell culture substrate has a layer comprising a hydrophilic polymer on the surface thereof, and
the region (A) is a region in which a part of the layer comprising the hydrophilic polymer is decomposed or modified by at least one treatment selected from a group consisting of plasma treatment, ultraviolet treatment, and corona discharge treatment.

21. The method for inducing differentiation according to any one of claims 16 to 20, wherein
in the step (3-1), the cell density when seeding the pluripotent stem cell is 1.0 × 10² to 5.0 × 10⁵ cells/cm², based on the area of the culture surface of the cell culture substrate.

22. The method for inducing differentiation according to any one of claims 16 to 21, wherein
a shape of the aggregate is a hemispherical shape.

23. The method for inducing differentiation method according to any one of claims 16 to 22, wherein
the differentiation-inducing factor is at least one selected from a group consisting of a TGF-β inhibitor, an ATP competitive inhibitor, and a GSK3 inhibitor.

24. A kit for inducing differentiation into a tridermic cell, comprising a cell culture substrate comprising a region (A) below and a region (B) below:
(A) an island-shaped region with an area of 0.001 to 5 mm² having cell adhesiveness and cell proliferation; and
(B) a region adjacent to the region (A) and having no cell adhesiveness or having no cell proliferation.
